# EUROPEAN PATENT APPLICATION

(11) **EP 4 822 022 A1**
(43) Date of publication of application: **14.10.2026**
(21) Application number: 24899648.0
(22) Date of filing: 22.11.2024
(51) Int. Cl.: C12N 15/113, A61P 15/00, A61P 17/10, A61K 8/60, A61Q 7/00, A61K 31/712

(54) **GROUP OF ANTISENSE OLIGONUCLEOTIDES THAT SPECIFICALLY INHIBIT ANDROGEN RECEPTOR, AND USE THEREOF**

(30) Priority: 05.12.2023 CN 202311654822
(71) Applicant: Lnctac Co., Ltd., Chengdu, Sichuan 610213 (CN)
(72) Inventor: CHEN, Haodong, Chengdu, Sichuan 610213 (CN); ZHANG, Rui, Chengdu, Sichuan 610213 (CN); LI, Wanwan, Chengdu, Sichuan 610213 (CN); ZHANG, Hailin, Chengdu, Sichuan 610213 (CN); ZHU, Yijun, Chengdu, Sichuan 610213 (CN); ZHANG, Xinghe, Chengdu, Sichuan 610213 (CN); ZHANG, Dan, Chengdu, Sichuan 610213 (CN); JIANG, Xiaobing, Chengdu, Sichuan 610213 (CN); LI, Yan, Chengdu, Sichuan 610213 (CN); ZHU, Honglin, Chengdu, Sichuan 610213 (CN); KOU, Junchuang, Chengdu, Sichuan 610213 (CN); WU, Lina, Chengdu, Sichuan 610213 (CN); ZHENG, Xin, Chengdu, Sichuan 610213 (CN); LI, Jun, Chengdu, Sichuan 610213 (CN); ZHANG, Lei, Chengdu, Sichuan 610213 (CN); DU, Jia, Chengdu, Sichuan 610213 (CN); GRIMM, Maximilian, Chengdu, Sichuan 610213 (CN); XU, Xiaoxue, Chengdu, Sichuan 610213 (CN); NIU, Zhenfeng, Chengdu, Sichuan 610213 (CN); LIU, Yu, Chengdu, Sichuan 610213 (CN); ZHANG, Jinyang, Chengdu, Sichuan 610213 (CN); HUANG, Xifang, Chengdu, Sichuan 610213 (CN); HUANG, Tingting, Chengdu, Sichuan 610213 (CN); YUAN, Chong, Chengdu, Sichuan 610213 (CN)
(74) Representative: KATZAROV S.A.
(86) International application number: PCT/CN2024/133958
(87) International publication number: WO 2025/119007

(57) **Abstract**

A group of antisense oligonucleotides that specifically inhibit an androgen receptor, and the use thereof. The antisense oligonucleotides have a length of 14-22 bases. The antisense oligonucleotides (ASO) have the following uses:
(1) preparing a preparation that inhibits the expression level of an AR protein; and
(2) preparing a drug or a pharmaceutical composition for treating androgen-induced alopecia; or
(3) preparing a drug or a pharmaceutical composition for treating androgen-induced acne; or
(4) inhibiting the expression or activation of the AR protein; and
(5) treating diseases caused by the overexpression or excessive activation of the AR protein, wherein the diseases include, but are not limited to, tumors, alopecia or acne caused by the overexpression or excessive activation of the AR protein.

## Description

The present disclosure claims the right of priority for Chinese patent application no. 202311654822.7, entitled "GROUP OF ANTISENSE OLIGONUCLEOTIDES THAT SPECIFICALLY INHIBIT ANDROGEN RECEPTOR, AND USE THEREOF" and filed to the China Patent Office on Dec. 5, 2023, which is incorporated herein by reference in its entirety.

### Technical Field

The present disclosure relates to the field of biotechnology, and specifically, to a group of antisense oligonucleotides that specifically inhibit the androgen receptor, and use thereof.

### Background

The epidemiological population affected by hair loss is growing, with androgenetic alopecia being the most prevalent form. The incidence rate among men aged 50 is 50%, and the lifetime prevalence is 90%; among women, the lifetime prevalence is 50%. However, current treatments are limited by a lack of options, poor efficacy, and significant side effects. Novel nucleic acid drugs represent a potential therapeutic approach for this type of skin condition. Since existing nucleic acid delivery systems have not yet been successfully applied to the treatment of skin conditions such as hair loss, the development of new transdermal delivery systems for nucleic acid drugs (and even protein-based and small-molecule drugs) could expand the therapeutic options for skin conditions and holds significant importance for the development of treatments for hair loss.

The androgen receptor (AR) is a nuclear receptor that, when inactive, resides in the cytoplasm, where it forms a complex with heat shock proteins and other chaperone proteins in a dimeric state. Upon binding to androgens, the complex dissociates, the AR dimer is phosphorylated, binds to other regulatory proteins, and then translocates into the cell nucleus. Subsequently, the DNA-binding domain of AR binds to DNA sequences containing androgen response elements (ARE; most typically TGTTCT), transcribing downstream genes and thereby regulating the functions of the reproductive, immune, and endocrine systems. It is worth noting that AR can only bind to testosterone and dihydrotestosterone (DHT, a highly active metabolite converted by type II 5α-reductase) within the dermal papilla cells of hair follicles on the scalp, thereby exerting its effects. These effects include shortening the hair growth phase, miniaturising or even atrophying the hair follicles, and ultimately causing hair loss.

In recent years, the development of AR-targeted drugs has focused on small-molecule inhibitors or agonists for oncological applications, which is a highly competitive field overall. By contrast, nucleic acid-based therapies that target this receptor to treat androgenetic alopecia and acne are still in the early stages of development. However, research interest in this area is growing and the potential for development is significant.

This disclosure is based on this.

### Summary of the Invention

In a first aspect, the present disclosure provides a group of antisense oligonucleotides (ASO) that specifically inhibit the androgen receptor (AR), wherein the ASO are 14 to 22 bases in length, the target gene is AR mRNA, and the ASO specifically hybridise to specific regions of the target gene, wherein the starting points of the specific regions are located at:
(1) 5'UTR region: 132-144, 164-167, 191-229, 266-298;
(2) Exon 1: 1199-1201, 1591-1610, 1892, 2209-2210, 2306, 2572-2574;
(3) Exon 2: 2777-2782;
(4) Exon 2-Exon 3: 2878-2882;
(5) Exon 3: 2981-2983;
(6) Exon 4: 3113-3116;
(7) Exon 5: 3397-3401;
(8) Exon 5-Exon 6: 3432-3443;
(9) Exon 6: 3464, 3505-3506;
(10) Exon 6-Exon 7: 3563-3573;
(11) Exon 7: 3612, 3614-3616, 3618/3619, 3627, 3629, 3631, 3633;
(12) Exon 8: 3749-3759, 3818-3863;
(13) 3'UTR region: 7346-7354, 7394, 8139, 8418-8423, 10358-10363, 10386-10425;

The AR target gene is designated as: ENST00000374690.9, comprises the sequence as shown in SEQ ID NO. 63.

Furthermore, the ASO comprises chemical modifications; preferably, the chemical modifications include: thiol groups on the phosphate ester bonds, methoxyethyl modification at the 2'-position of the base (2'-MOE modification), and 5-methyl modification of cytosine.

More preferably, the modification comprises: mono-sulfur modification of the phosphate bonds of all the nucleotides in the ASO sequence, 2'-MOE modification on the same number of 3 to 5 bases at both the 3' and 5' ends, respectively, and 5-methyl modification of all cytosines.

Furthermore, the ASO is selected from the oligonucleotides shown in any of the sequences listed in the table below;

| ID | SEQ ID NO | Sequence | Target Region startPos(+) | Target Region |
|---|---|---|---|---|
| 150 | 1 | TGGCGTGGTGCGTCCC | 134 | 5UTR EXON1 |
| 13 | 2 | TGGGGCTGGCGTGGTGCGTCCC | 134 | 5UTR EXON1 |
| 161 | 3 | CTGGCGTGGTGCGTCC | 135 | 5UTR EXON1 |
| 134 | 4 | GCTGGCGTGGTGCGTCC | 135 | 5UTR EXON1 |
| 162 | 5 | GCTGGCGTGGTGCGTC | 136 | 5UTR EXON1 |
| 157 | 6 | GGCTGGCGTGGTGCGT | 137 | 5UTR EXON1 |
| 18 | 7 | GGCTGGGGCTGGCGTGGTGCGT | 137 | 5UTR EXON1 |
| 169 | 8 | GGGCTGGCGTGGTGCG | 138 | 5UTR EXON1 |
| 178 | 9 | GGGGCTGGCGTGGTGC | 139 | 5UTR EXON1 |
| 180 | 10 | CTGGGGCTGGCGTGGT | 141 | 5UTR EXON1 |
| 106 | 11 | GGCTGGGGCTGGCGTGGT | 141 | 5UTR EXON1 |
| 182 | 12 | GCTGGGGCTGGCGTGG | 142 | 5UTR EXON1 |
| 181 | 13 | GGCTGGGGCTGGCGTG | 143 | 5UTR EXON1 |
| 183 | 14 | GGGCTGGGGCTGGCGT | 144 | 5UTR EXON1 |
| 153 | 15 | CGTTGGCTGTCGCTGG | 164 | 5UTR EXON1 |
| 89 | 16 | GGCGTTGGCTGTCGCTGG | 164 | 5UTR EXON1 |
| 131 | 17 | GCGTTGGCTGTCGCTGG | 164 | 5UTR EXON1 |
| 164 | 18 | GCGTTGGCTGTCGCTG | 165 | 5UTR EXON1 |
| 120 | 19 | GGCGTTGGCTGTCGCTG | 165 | 5UTR EXON1 |
| 111 | 20 | AGGCGTTGGCTGTCGCT | 166 | 5UTR EXON1 |
| 151 | 21 | AGGCGTTGGCTGTCGC | 167 | 5UTR EXON1 |
| 163 | 22 | GCTTCGAAGCCGCCGC | 191 | 5UTR EXON1 |
| 156 | 23 | GGCTTCGAAGCCGCCG | 192 | 5UTR EXON1 |
| 155 | 24 | CGGCTTCGAAGCCGCC | 193 | 5UTR EXON1 |
| 152 | 25 | GGGCGGCGGCTTCGAA | 199 | 5UTR EXON1 |
| 176 | 26 | AGGAAAGGGCAGCTCC | 215 | 5UTR EXON1 |
| 177 | 27 | GAGGAAAGGGCAGCTC | 216 | 5UTR EXON1 |
| 174 | 28 | CGAAGAGGAAAGGGCA | 220 | 5UTR EXON1 |
| 167 | 29 | CCGAAGAGGAAAGGGC | 221 | 5UTR EXON1 |
| 166 | 30 | ACCGAAGAGGAAAGGG | 222 | 5UTR EXON1 |
| 173 | 31 | CACCGAAGAGGAAAGG | 223 | 5UTR EXON1 |
| 165 | 32 | TCACCGAAGAGGAAAG | 224 | 5UTR EXON1 |
| 172 | 33 | TTCACCGAAGAGGAAA | 225 | 5UTR EXON1 |
| 94 | 34 | ACTTCACCGAAGAGGAAA | 225 | 5UTR EXON1 |
| 158 | 35 | CTTCACCGAAGAGGAA | 226 | 5UTR EXON1 |
| 159 | 36 | ACTTCACCGAAGAGGA | 227 | 5UTR EXON1 |
| 81 | 37 | AAACTTCACCGAAGAGGA | 227 | 5UTR EXON1 |
| 154 | 38 | AACTTCACCGAAGAGG | 228 | 5UTR EXON1 |
| 149 | 39 | AAACTTCACCGAAGAG | 229 | 5UTR EXON1 |
| 170 | 40 | ACTTTCCTTGCTTCCT | 267 | 5UTR EXON1 |
| 179 | 41 | CACTTTCCTTGCTTCC | 268 | 5UTR EXON1 |
| 175 | 42 | GGACAAAGGCAGCCGT | 297 | 5UTR EXON1 |
| 91 | 43 | GAGGACAAAGGCAGCCGT | 297 | 5UTR EXON1 |
| 168 | 44 | AGGACAAAGGCAGCCG | 298 | 5UTR EXON1 |
| 237 | 45 | CGCGCACGCTCTGGAACAGA | 1201 | EXON1 |
| 194 | 46 | GAAAGTTGTAGTAGTCGCGA | 2209 | EXON1 |
| 271 | 47 | AAGTTGTAGTAGTCGCGA | 2209 | EXON1 |
| 204 | 48 | GGAAAGTTGTAGTAGTCGCG | 2210 | EXON1 |
| 272 | 49 | AAAGTTGTAGTAGTCGCG | 2210 | EXON1 |
| 250 | 50 | TGCATGCGGTACTCATTGAA | 3437 | EXON5_EXON6 |
| 193 | 51 | GTGCATGCGGTACTCATTGA | 3438 | EXON5_EXON6 |
| 219 | 52 | TGCCAGTGAACATACATAAA | 7346 | EXON8 3UTR |
| 192 | 53 | GTGCCAGTGAACATACATAA | 7347 | EXON8 3UTR |
| 289 | 54 | TGCCAGTGAACATACATA | 7348 | EXON8 3UTR |
| 285 | 55 | TAGTGCCAGTGAACATAC | 7351 | EXON8 3UTR |
| 4 | 56 | TTTTTAGTGCCAGTGAACAT | 7353 | EXON8 3UTR |
| 300 | 57 | TTTTAGTGCCAGTGAACA | 7354 | EXON8 3UTR |
| 206 | 58 | GAGGCACTAATGCTTGCTCC | 8139 | EXON8 3UTR |
| 5 | 59 | AACAGAACACTAGCGCTTGG | 10358 | EXON8 3UTR |
| 3 | 60 | GAACAGAACACTAGCGCTTG | 10359 | EXON8 3UTR |
| 241 | 61 | AGAGAACAGAACACTAGCGC | 10362 | EXON8 3UTR |
| 280 | 62 | GAGAACAGAACACTAGCG | 10363 | EXON8 3UTR |

Preferably, in the ASO described in the table, all nucleotide phosphate bonds are mono-sulfated, both the 3' and 5' ends have same number of 4-5 bases modified with MOE, and all cytosines modified with 5-methyl groups.

More preferably, the ASO has a sequence motif pattern of 4-8-4, 4-9-4, 5-8-5, 5-10-5, or 5-12-5.

In a second aspect, the present disclosure further discloses the following uses of the aforementioned antisense oligonucleotides (ASO) or their modified form:
(1) Preparing a formulation to inhibit the expression of the AR protein;
(2) Preparing a drug or pharmaceutical composition for treating androgen-induced hair loss; or
(3) Preparing a drug or pharmaceutical composition for treating androgen-induced acne; or
(4) Inhibiting the expression or activation of the AR protein;
(5) Treating diseases caused by over-expression or excessive activation of the AR protein, wherein the diseases include but are not limited to: tumors, alopecia, or acne caused by overexpression or excessive activation of the AR protein; preferably, the tumor is prostate cancer.

The drug or drug composition comprises a therapeutically effective amount of the aforementioned antisense oligonucleotides (ASO) or their modified form, together with the necessary pharmaceutical excipients or delivery carriers.

In a third aspect, the present disclosure further discloses a pharmaceutical composition comprising the aforementioned antisense oligonucleotides (ASO) or their modified form, wherein the pharmaceutical composition comprises: a therapeutically effective amount of the antisense oligonucleotides (ASO) or their modified form, and necessary pharmaceutical excipients or delivery carriers.

Preferably, the pharmaceutical composition is a transdermal formulation.

In a fourth aspect, the present disclosure further discloses a topical formulation containing the aforementioned antisense oligonucleotides (ASO) or their modified form, wherein the formulation comprises:
(1) the aforementioned antisense oligonucleotide (ASO) or their modified form: 1-50 mg/mL; preferably 2-20 mg/mL; more preferably 4-10 mg/mL;
(2) sodium 8-(2-hydroxybenzoylamino) octanoate (SNAC): 5-50 mg/mL, preferably 30 mg/mL;
(3) dimethyl isosorbide (DMI): 5-60 mg/mL, preferably 40 mg/mL;
(4) vitamin E polyethylene glycol succinate (TPGS): 2-20 mg/mL, preferably 10 mg/mL;
(5) vitamin C: 2-20 mg/mL, preferably 10 mg/mL

Preferably, the chemically form of the antisense oligonucleotide (ASO) in the topical formulation is
(1) LT-0390 (ASO ID. 289):
   T*G*C*C*A*g*t*g*a*a*c*a*t*A*C*A*T*A*HSA, modified as follows:
   *: phosphorothioate inter-nucleoside linkage;
   A, G, C, T: 2'-O-methoxyethyl; (2'-MOE);
   a, g, c, t: 2'-deoxy;
   all C or c base is 5-methyl-modified cytosine;
   HSA is: 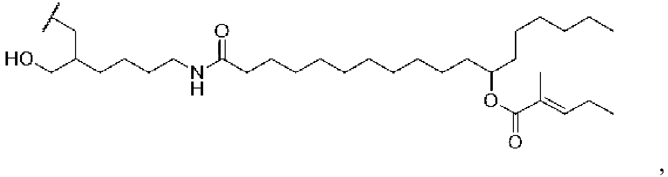 or
(2) LT-0449 (ASO ID. 289):
   T*G*CCA*g*t*g*a*a*c*a*t*ACA*T*A*HSA, modified as follows:
   *: phosphorothioate inter-nucleoside linkage;
   A, G, C, T: 2'-O-methoxyethyl; (2'-MOE);
   a, g, c, t: 2'-deoxy;
   all C or c base is 5-methyl-modified cytosine;
   HSA is: 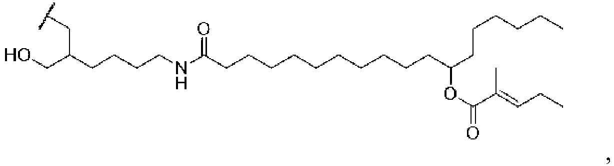 or
(3) LT-0410 (ASO ID. 204) :
   G*G*A*A*A*g*t*t*g*t*a*g*t*a*g*T*C*G*C*G*RA, modified as follows:
   *: phosphorothioate inter-nucleoside linkage;
   A, G, C, T: 2'-O-methoxyethyl; (2'-MOE);
   a, g, t: 2'-deoxy;
   all C base is 5-methyl-modified cytosine;
   RA is:

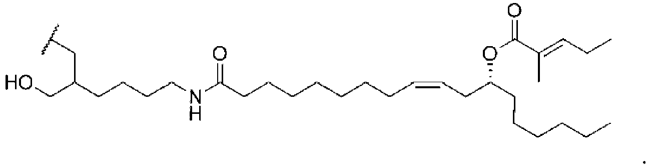

In a fifth aspect, the present disclosure further discloses a method for preparing the aforementioned topical formulation, includes the following steps:
(1) Oxygen equilibration: take purified water and equilibrate the dissolved oxygen in the water by purging it with nitrogen at a pressure of 0.1-0.2 MPa;
(2) Dissolving excipients and adjusting pH: add SNAC, DMI, vitamin C, and TPGS to an appropriate amount of oxygen-equilibrated water and stir until dissolved; adjust the pH to 7.5-9.0, and re-inject nitrogen to re-equilibrate the dissolved oxygen;
(3) Dissolving of the Active Pharmaceutical Ingredient (API): add the API (the aforementioned ASO or its modified form), dissolve it, and make up the volume with oxygen-equilibrated water; after making up the volume, nitrogen gas is introduced again to re-equilibrate the dissolved oxygen, resulting in the ASO/ASO-modified topical formulation.
and the optional step (4) Filling and Labeling: transfer the formulation to borosilicate glass vials, purge with nitrogen, seal with a rubber stopper and aluminum cap, crimp the cap, and apply a label.

In a sixth aspect, the present disclosure further discloses the following uses of the aforementioned topical formulation:
(1) preparing a drug or pharmaceutical composition for treating male pattern hair loss; or
(2) preparing a drug or pharmaceutical composition for treating acne; or
(3) treating hair loss or acne caused by the overexpression or excessive activation of the AR protein.

### Brief Description of the Figures

FIG. 1 After treating cells with the antisense oligonucleotides (ASO), Western blot (WB) analysis was performed to assess the inhibitory effect on AR protein expression.
FIG. 2 After treating cells with the antisense oligonucleotides (ASO), Western blot (WB) analysis was performed to assess the inhibitory effect on AR protein expression.
FIG. 3 The stability of the ASO in serum was assessed using electrophoresis.
FIG. 4 The expression of mRNA from cytokines associated with inflammatory responses was detected following treatment of cells with the antisense oligonucleotides (ASO).

### Detailed Description of Embodiments

### Example 1 Design, Synthesis, and Modification of ASO Targeting the AR Gene

The AR gene (ENST00000374690.9) from the Ensembl database was selected as the target gene; the sequence of the target gene is shown in SEQ ID NO. 63,

SEQ ID NO.63:
AGCGCCCCCTCCGAGATCCCGGGGAGCCAGCTTGCTGGGAGAGCGGACGGTCCGGAGCAA
GCCCAGAGGCAGAGGAGGCGACAGAGGAAAAAGGGCCGAGCTAGCCGCTCCAGTGCTGTACAGG
AGCCGAAGGGACGCACCACGCCAGCCCCAGCCCGGCTCCAGCGACAGCCAACGCCTCTTGCAGCGC
GGCGGCTTCGAAGCCGCCGCCCGGAGCTGCCCTTTCCTCTTCGGTGAAGTTTTTAAAAGCTGCTAAAG
ACTCGGAGGAAGCAAGGAAAGTGCCTGGTAGGACTGACGGCTGCCTTTGTCCTCCTCCTCTCCACCC
CGCCTCCCCCCACCCTGCCTTCCCCCCCTCCCCCGTCTTCTCTCCCGCAGCTGCCTCAGTCGGCTACTC
TCAGCCAACCCCCCTCACCACCCTTCTCCCCACCCGCCCCCCCGCCCCCGTCGGCCCAGCGCTGCCAG
CCCGAGTTTGCAGAGAGGTAACTCCCTTTGGCTGCGAGCGGGCGAGCTAGCTGCACATTGCAAAGAA
GGCTCTTAGGAGCCAGGCGACTGGGGAGCGGCTTCAGCACTGCAGCCACGACCCGCCTGGTTAGGCT
GCACGCGGAGAGAACCCTCTGTTTTCCCCCACTCTCTCTCCACCTCCTCCTGCCTTCCCCACCCCGAG
TGCGGAGCCAGAGATCAAAAGATGAAAAGGCAGTCAGGTCTTCAGTAGCCAAAAAACAAAACAAAC
AAAAACAAAAAAGCCGAAATAAAAGAAAAAGATAATAACTCAGTTCTTATTTGCACCTACTTCAGTG
GACACTGAATTTGGAAGGTGGAGGATTTTGTTTTTTTCTTTTAAGATCTGGGCATCTTTTGAATCTACC
CTTCAAGTATTAAGAGACAGACTGTGAGCCTAGCAGGGCAGATCTTGTCCACCGTGTGTCTTCTTCTG

CACGAGACTTTGAGGCTGTCAGAGCGCTTTTTGCGTGGTTGCTCCCGCAAGTTTCCTTCTCTGGAGCT
TCCCGCAGGTGGGCAGCTAGCTGCAGCGACTACCGCATCATCACAGCCTGTTGAACTCTTCTGAGCA
AGAGAAGGGGAGGCGGGGTAAGGGAAGTAGGTGGAAGATTCAGCCAAGCTCAAGGATGGAAGTGC
AGTTAGGGCTGGGAAGGGTCTACCCTCGGCCGCCGTCCAAGACCTACCGAGGAGCTTTCCAGAATCT
GTTCCAGAGCGTGCGCGAAGTGATCCAGAACCCGGGCCCCAGGCACCCAGAGGCCGCGAGCGCAGC
ACCTCCCGGCGCCAGTTTGCTGCTGCTGCAGCAGCAGCAGCAGCAGCAGCAGCAGCAGCAGCAGCA
GCAGCAGCAGCAGCAGCAGCAGCAGCAGCAAGAGACTAGCCCCAGGCAGCAGCAGCAGCAGCAGG
GTGAGGATGGTTCTCCCCAAGCCCATCGTAGAGGCCCCACAGGCTACCTGGTCCTGGATGAGGAACA
GCAACCTTCACAGCCGCAGTCGGCCCTGGAGTGCCACCCCGAGAGAGGTTGCGTCCCAGAGCCTGGA
GCCGCCGTGGCCGCCAGCAAGGGGCTGCCGCAGCAGCTGCCAGCACCTCCGGACGAGGATGACTCA
GCTGCCCCATCCACGTTGTCCCTGCTGGGCCCCACTTTCCCCGGCTTAAGCAGCTGCTCCGCTGACCT
TAAAGACATCCTGAGCGAGGCCAGCACCATGCAACTCCTTCAGCAACAGCAGCAGGAAGCAGTATC
CGAAGGCAGCAGCAGCGGGAGAGCGAGGGAGGCCTCGGGGGCTCCCACTTCCTCCAAGGACAATTA
CTTAGGGGGCACTTCGACCATTTCTGACAACGCCAAGGAGTTGTGTAAGGCAGTGTCGGTGTCCATG
GGCCTGGGTGTGGAGGCGTTGGAGCATCTGAGTCCAGGGGAACAGCTTCGGGGGGATTGCATGTAC
GCCCCACTTTTGGGAGTTCCACCCGCTGTGCGTCCCACTCCTTGTGCCCCATTGGCCGAATGCAAAGG
TTCTCTGCTAGACGACAGCGCAGGCAAGAGCACTGAAGATACTGCTGAGTATTCCCCTTTCAAGGGA
GGTTACACCAAAGGGCTAGAAGGCGAGAGCCTAGGCTGCTCTGGCAGCGCTGCAGCAGGGAGCTCC
GGGACACTTGAACTGCCGTCTACCCTGTCTCTCTACAAGTCCGGAGCACTGGACGAGGCAGCTGCGT
ACCAGAGTCGCGACTACTACAACTTTCCACTGGCTCTGGCCGGACCGCCGCCCCCTCCGCCGCCTCCC
CATCCCCACGCTCGCATCAAGCTGGAGAACCCGCTGGACTACGGCAGCGCCTGGGCGGCTGCGGCG
CGCAGTGCCGCTATGGGGACCTGGCGAGCCTGCATGGCGCGGGTGCAGCGGGACCCGGTTCTGGGTC
ACCCTCAGCCGCCGCTTCCTCATCCTGGCACACTCTCTTCACAGCCGAAGAAGGCCAGTTGTATGGAC
CGTGTGGTGGTGGTGGGGGTGGTGGCGGCGGCGGCGGCGGCGGCGGCGGCGGCGGCGGCGGCGGCG
GCGGCGGCGAGGCGGGAGCTGTAGCCCCCTACGGCTACACTCGGCCCCCTCAGGGGCTGGCGGGCC
AGGAAAGCGACTTCACCGCACCTGATGTGTGGTACCCTGGCGGCATGGTGAGCAGAGTGCCCTATCC
CAGTCCCACTTGTGTCAAAAGCGAAATGGGCCCCTGGATGGATAGCTACTCCGGACCTTACGGGGAC
ATGCGTTTGGAGACTGCCAGGGACCATGTTTTGCCCATTGACTATTACTTTCCACCCCAGAAGACCTG
CCTGATCTGTGGAGATGAAGCTTCTGGGTGTCACTATGGAGCTCTCACATGTGGAAGCTGCAAGGTC
TTCTTCAAAAGAGCCGCTGAAGGGAAACAGAAGTACCTGTGCGCCAGCAGAAATGATTGCACTATTG
ATAAATTCCGAAGGAAAAATTGTCCATCTTGTCGTCTTCGGAAATGTTATGAAGCAGGGATGACTCT
GGGAGCCCGGAAGCTGAAGAAACTTGGTAATCTGAAACTACAGGAGGAAGGAGAGGCTTCCAGCAC
CACCAGCCCCACTGAGGAGACAACCCAGAAGCTGACAGTGTCACACATTGAAGGCTATGAATGTCA
GCCCATCTTTCTGAATGTCCTGGAAGCCATTGAGCCAGGTGTAGTGTGTGCTGGACACGACAACAAC
CAGCCCGACTCCTTTGCAGCCTTGCTCTCTAGCCTCAATGAACTGGGAGAGAGACAGCTTGTACACG
TGGTCAAGTGGGCCAAGGCCTTGCCTGGCTTCCGCAACTTACACGTGGACGACCAGATGGCTGTCAT
TCAGTACTCCTGGATGGGGCTCATGGTGTTTGCCATGGGCTGGCGATCCTTCACCAATGTCAACTCCA
GGATGCTCTACTTCGCCCCTGATCTGGTTTTCAATGAGTACCGCATGCACAAGTCCCGGATGTACAGC
CAGTGTGTCCGAATGAGGCACCTCTCTCAAGAGTTTGGATGGCTCCAAATCACCCCCCAGGAATTCC
TGTGCATGAAAGCACTGCTACTCTTCAGCATTATTCCAGTGGATGGGCTGAAAAATCAAAAATTCTTT
GATGAACTTCGAATGAACTACATCAAGGAACTCGATCGTATCATTGCATGCAAAAGAAAAAATCCCA
CATCCTGCTCAAGACGCTTCTACCAGCTCACCAAGCTCCTGGACTCCGTGCAGCCTATTGCGAGAGA
GCTGCATCAGTTCACTTTTGACCTGCTAATCAAGTCACACATGGTGAGCGTGGACTTTCCGGAAATGA
TGGCAGAGATCATCTCTGTGCAAGTGCCCAAGATCCTTTCTGGGAAAGTCAAGCCCATCTATTTCCAC
ACCCAGTGAAGCATTGGAAACCCTATTTCCCCACCCCAGCTCATGCCCCCTTTCAGATGTCTTCTGCC

TGTTATAACTCTGCACTACTCCTCTGCAGTGCCTTGGGGAATTTCCTCTATTGATGTACAGTCTGTCAT
GAACATGTTCCTGAATTCTATTTGCTGGGCTTTTTTTTCTCTTTCTCTCCTTTCTTTTTCTTCTTCCCTC
CCTATCTAACCCTCCCATGGCACCTTCAGACTTTGCTTCCCATTGTGGCTCCTATCTGTGTTTTGAATG
GTGTTGTATGCCTTTAAATCTGTGATGATCCTCATATGGCCCAGTGTCAAGTTGTGCTTGTTTACAGC
ACTACTCTGTGCCAGCCACACAAACGTTTACTTATCTTATGCCACGGGAAGTTTAGAGAGCTAAGATT
ATCTGGGGAAATCAAAACAAAAACAAGCAAACAAAAAAAAAAAGCAAAAACAAAACAAAAAATAA
GCCAAAAAACCTTGCTAGTGTTTTTCCTCAAAAATAAATAAATAAATAAATACGTACATACA
TACACACATACATACAAACATATAGAAATCCCCAAAGAGGCCAATAGTGACGAGAAGGTGAAAATT
GCAGGCCCATGGGGAGTTACTGATTTTTCATCTCCTCCCTCCACGGGAGACTTTATTTCTGCCAAT
GGCTATTGCCATTAGAGGGCAGAGTGACCCCAGAGCTGAGTTGGGCAGGGGGGTGGACAGAGAGGA
GAGGACAAGGAGGGCAATGGAGCATCAGTACCTGCCCACAGCCTTGGTCCCTGGGGGCTAGACTGC
TCAACTGTGGAGCAATTCATTATACTGAAAATGTGCTTGTTGTTGAAAATTTGTCTGCATGTTAATGC
CTCACCCCCAAACCCTTTTCTCTCTCACTCTCTGCCTCCAACTTCAGATTGACTTTCAATAGTTTTTCT
AAGACCTTTGAACTGAATGTTCTCTTCAGCCAAAACTTGGCGACTTCCACAGAAAAGTCTGACCACT
GAGAAGAAGGAGAGCAGAGATTTAACCCTTTGTAAGGCCCCATTTGGATCCAGGTCTGCTTTCTCAT
GTGTGAGTCAGGGAGGAGCTGGAGCCAGAGGAGAAGAAAATGATAGCTTGGCTGTTCTCCTGCTTA
GGACACTGACTGAATAGTTAAACTCTCACTGCCACTACCTTTTCCCCACCTTTAAAAGACCTGAATGA
AGTTTTCTGCCAAACTCCGTGAAGCCACAAGCACCTTATGTCCTCCCTTCAGTGTTTTGTGGGCCTGA
ATTTCATCACACTGCATTTCAGCCATGGTCATCAAGCCTGTTTGCTTCTTTTGGGCATGTTCACAGATT
CTCTGTTAAGAGCCCCCACCACCAAGAAGGTTAGCAGGCCAACAGCTCTGACATCTATCTGTAGATG
CCAGTAGTCACAAAGATTTCTTACCAACTCTCAGATCGCTGGAGCCCTTAGACAAACTGGAAAGAAG
GCATCAAAGGGATCAGGCAAGCTGGGCGTCTTGCCCTTGTCCCCCAGAGATGATACCCTCCCAGCAA
GTGGAGAAGTTCTCACTTCCTTCTTTAGAGCAGCTAAAGGGGCTACCCAGATCAGGGTTGAAGAGAA
AACTCAATTACCAGGGTGGGAAGAATGAAGGCACTAGAACCAGAAACCCTGCAAATGCTCTTCTTGT
CACCCAGCATATCCACCTGCAGAAGTCATGAGAAGAGAGAAGGAACAAAGAGGAGACTCTGACTAC
TGAATTAAAATCTTCAGCGGCAAAGCCTAAAGCCAGATGGACACCATCTGGTGAGTTTACTCATCAT
CCTCCTCTGCTGCTGATTCTGGGCTCTGACATTGCCCATACTCACTCAGATTCCCCACCTTTGTTGCTG
CCTCTTAGTCAGAGGGAGGCCAAACCATTGAGACTTTCTACAGAACCATGGCTTCTTTCGGAAAGGT
CTGGTTGGTGTGGCTCCAATACTTTGCCACCCATGAACTCAGGGTGTGCCCTGGGACACTGGTTTTAT
ATAGTCTTTTGGCACACCTGTGTTCTGTTGACTTCGTTCTTCAAGCCCAAGTGCAAGGGAAAATGTCC
ACCTACTTTCTCATCTTGGCCTCTGCCTCCTTACTTAGCTCTTAATCTCATCTGTTGAACTCAAGAAAT
CAAGGGCCAGTCATCAAGCTGCCCATTTTAATTGATTCACTCTGTTTGTTGAGAGGATAGTTTCTGAG
TGACATGATATGATCCACAAGGGTTTCCTTCCCTGATTTCTGCATTGATATTAATAGCCAAACGAACT
TCAAAACAGCTTTAAATAACAAGGGAGAGGGGAACCTAAGATGAGTAATATGCCAATCCAAGACTG
CTGGAGAAAACTAAAGCTGACAGGTTCCCTTTTTGGGGTGGGATAGACATGTTCTGGTTTTCTTTATT
ATTACACAATCTGGCTCATGTACAGGATCACTTTTAGCTGTTTTAAACAGAAAAAAATATCCACCACT
CTTTTCAGTTACACTAGGTTACATTTTAATAGGTCCTTTACATCTGTTTTGGAATGATTTTCATCTTTT
GTGATACACAGATTGAATTATATCATTTTCATATCTCTCCTTGTAAATACTAGAAGCTCTCCTTTACAT
TTCTCTATCAAATTTTTCATCTTTATGGGTTTCCCAATTGTGACTCTTGTCTTCATGAATATATGTTTTT
CATTTGCAAAAGCCAAAAATCAGTGAAACAGCAGTGTAATTAAAAGCAACAACTGGATTACTCCAA
ATTTCCAAATGACAAAACTAGGGAAAAATAGCCTACACAAGCCTTTAGGCCTACTCTTTCTGTGCTTG
GGTTTGAGTGAACAAAGGAGATTTTAGCTTGGCTCTGTTCTCCCATGGATGAAAGGAGGAGGATTTT
TTTTTTCTTTTGGCCATTGATGTTCTAGCCAATGTAATTGACAGAAGTCTCATTTTGCATGCGCTCTGC
TCTACAAACAGAGTTGGTATGGTTGGTATACTGTACTCACCTGTGAGGGACTGGCCACTCAGACCCA
CTTAGCTGGTGAGCTAGAAGATGAGGATCACTCACTGGAAAAGTCACAAGGACCATCTCCAAACAA

GTTGGCAGTGCTCGATGTGGACGAAGAGTGAGGAAGAGAAAAAGAAGGAGCACCAGGGAGAAGGC
TCCGTCTGTGCTGGGCAGCAGACAGCTGCCAGGATCACGAACTCTGTAGTCAAAGAAAAGAGTCGTG
TGGCAGTTTCAGCTCTCGTTCATTGGGCAGCTCGCCTAGGCCCAGCCTCTGAGCTGACATGGGAGTTG
TTGGATTCTTTGTTTCATAGCTTTTTCTATGCCATAGGCAATATTGTTGTTCTTGGAAAGTTTATTATTT
TTTTAACTCCCTTACTCTGAGAAAGGGATATTTGAAGGACTGTCATATATCTTTGAAAAAGAAAAT
CTGTAATACATATATTTTTATGTATGTTCACTGGCACTAAAAATATAGAGAGCTTCATTCTGTCCTTT
GGGTAGTTGCTGAGGTAATTGTCCAGGTTGAAAAATAATGTGCTGATGCTAGAGTCCCTCTCTGTCCA
TACTCTACTTCTAAATACATATAGGCATACATAGCAAGTTTTATTTGACTTGTACTTTAAGAGAAAAT
ATGTCCACCATCCACATGATGCACAAATGAGCTAACATTGAGCTTCAAGTAGCTTCTAAGTGTTTGTT
TCATTAGGCACAGCACAGATGTGGCCTTTCCCCCCTTCTCTCCCTTGATATCTGGCAGGGCATAAAGG
CCCAGGCCACTTCCTCTGCCCCTTCCCAGCCCTGCACCAAAGCTGCATTTCAGGAGACTCTCTCCAGA
CAGCCCAGTAACTACCCGAGCATGGCCCCTGCATAGCCCTGGAAAAATAAGAGGCTGACTGTCTACG
AATTATCTTGTGCCAGTTGCCCAGGTGAGAGGGCACTGGGCCAAGGGAGTGGTTTTCATGTTTGACC
CACTACAAGGGGTCATGGGAATCAGGAATGCCAAAGCACCAGATCAAATCCAAAACTTAAAGTCAA
AATAAGCCATTCAGCATGTTCAGTTTCTTGGAAAAGGAAGTTTCTACCCCTGATGCCTTTGTAGGCAG
ATCTGTTCTCACCATTAATCTTTTTGAAAATCTTTTAAAGCAGTTTTTAAAAAGAGAGATGAAAGCAT
CACATTATATAACCAAAGATTACATTGTACCTGCTAAGATACCAAAATTCATAAGGGCAGGGGGGGA
GCAAGCATTAGTGCCTCTTTGATAAGCTGTCCAAAGACAGACTAAAGGACTCTGCTGGTGACTGACT
TATAAGAGCTTTGTGGGTTTTTTTTCCCTAATAATATACATGTTTAGAAGAATTGAAAATAATTTCG
GGAAAATGGGATTATGGGTCCTTCACTAAGTGATTTTATAAGCAGAACTGGCTTTCCTTTTCTCTAGT
AGTTGCTGAGCAAATTGTTGAAGCTCCATCATTGCATGGTTGGAAATGGAGCTGTTCTTAGCCACTGT
GTTTGCTAGTGCCCATGTTAGCTTATCTGAAGATGTGAAACCCTTGCTGATAAGGGAGCATTTAAAGT
ACTAGATTTTGCACTAGAGGGACAGCAGGCAGAAATCCTTATTTCTGCCCACTTTGGATGGCACAAA
AAGTTATCTGCAGTTGAAGGCAGAAAGTTGAAATACATTGTAAATGAATATTTGTATCCATGTTTCA
AAATTGAAATATATATATATATATATATATATATATATATATATATATATATAGTGTGTGTGTGTTCTGA
TAGCTTTAACTTTCTCTGCATCTTTATATTTGGTTCCAGATCACACCTGATGCCATGTACTTGTGAGAG
AGGATGCAGTTTTGTTTTGGAAGCTCTCTCAGAACAAACAAGACACCTGGATTGATCAGTTAACTAA
AAGTTTTCTCCCCTATTGGGTTTGACCCACAGGTCCTGTGAAGGAGCAGAGGGATAAAAAGAGTAGA
GGACATGATACATTGTACTTTACTAGTTCAAGACAGATGAATGTGGAAAGCATAAAAACTCAATGGA
ACTGACTGAGATTTACCACAGGGAAGGCCCAAACTTGGGGCCAAAAGCCTACCCAAGTGATTGACC
AGTGGCCCCCTAATGGGACCTGAGCTGTTGGAAGAAGAGAACTGTTCCTTGGTCTTCACCATCCTTGT
GAGAGAAGGGCAGTTTCCTGCATTGGAACCTGGAGCAAGCGCTCTATCTTTCACACAAATTCCCTCA
CCTGAGATTGAGGTGCTCTTGTTACTGGGTGTCTGTGTGCTGTAATTCTGGTTTTGGATATGTTCTGTA
AAGATTTTGACAAATGAAAATGTGTTTTTCTCTGTTAAAACTTGTCAGAGTACTAGAAGTTGTATCTC
TGTAGGTGCAGGTCCATTTCTGCCCACAGGTAGGGTGTTTTTCTTTGATTAAGAGATTGACACTTCTG
TTGCCTAGGACCTCCCAACTCAACCATTTCTAGGTGAAGGCAGAAAAATCCACATTAGTTACTCCTCT
TCAGACATTTCAGCTGAGATAACAAATCTTTTGGAATTTTTTCACCCATAGAAAGAGTGGTAGATATT
TGAATTTAGCAGGTGGAGTTTCATAGTAAAAACAGCTTTTGACTCAGCTTTGATTTATCCTCATTTGA
TTTGGCCAGAAAGTAGGTAATATGCATTGATTGGCTTCTGATTCCAATTCAGTATAGCAAGGTGCTAG
GTTTTTTCCTTTCCCCACCTGTCTCTTAGCCTGGGGAATTAAATGAGAAGCCTTAGAATGGGTGGCCC
TTGTGACCTGAAACACTTCCCACATAAGCTACTTAACAAGATTGTCATGGAGCTGCAGATTCCATTGC
CCACCAAAGACTAGAACACACACATATCCATACACCAAAGGAAAGACAATTCTGAAATGCTGTTTCT
CTGGTGGTTCCCTCTCTGGCTGCTGCCTCACAGTATGGGAACCTGTACTCTGCAGAGGTGACAGGCCA
GATTTGCATTATCTCACAACCTTAGCCCTTGGTGCTAACTGTCCTACAGTGAAGTGCCTGGGGGGTTG
TCCTATCCCATAAGCCACTTGGATGCTGACAGCAGCCACCATCAGAATGACCCACGCAAAAAAAAGA

AAAAAAAAATTAAAAAGTCCCCTCACAACCCAGTGACACCTTTCTGCTTTCCTCTAGACTGGAACAT
TGATTAGGGAGTGCCTCAGACATGACATTCTTGTGCTGTCCTTGGAATTAATCTGGCAGCAGGAGGG
AGCAGACTATGTAAACAGAGATAAAAATTAATTTTCAATATTGAAGGAAAAAAGAAATAAGAAGAG
AGAGAGAAAGAAAGCATCACACAAAGATTTCTTAAAAGAAACAATTTTGCTTGAAATCTCTTTAGA
TGGGGCTCATTTCTCACGGTGGCACTTGGCCTCCACTGGGCAGCAGGACCAGCTCCAAGCGCTAGTG
TTCTGTTCTCTTTTTGTAATCTTGGAATCTTTTGTTGCTCTAAATACAATTAAAAATGGCAGAAACTTG
TTTGTTGGACTACATGTGTGACTTTGGGTCTGTCTCTGCCTCTGCTTTCAGAAATGTCATCCATTGTGT
AAAATATTGGCTTACTGGTCTGCCAGCTAAAACTTGGCCACATCCCCTGTTATGGCTGCAGGATCGA
GTTATTGTTAACAAAGAGACCCAAGAAAAGCTGCTAATGTCCTCTTATCATTGTTGTTAATTTGTTAA
AACATAAAGAAATCTAAAATTTCA

A total of 7,650 antisense oligonucleotides (ASO) sequences targeting AR target genes were designed, and 349 ASO sequences were selected for synthesis and in vitro efficacy testing. The specific locations and information for these ASO are shown in Table 1.

Furthermore, the 349 ASO sequences were chemically modified as follows: all nucleotides in the sequence were modified with a single thiol group at the phospholipid bond, the 3' and 5' ends were each modified with 3-5 bases via MOE modification (methoxyethyl modification at the 2' position of the bases).

**Table 1: Detailed information on the ASO.**

| **EXON** | **Number of the ASO** | **Start Position** |
|---|---|---|
| EXON1 | 223 | 132-144, 164-167, 191-229, 266-298, 1199-1201, 1591-1610, 1892, 2209-2210, 2306, 2572-2574 |
| EXON2 | 6 | 2777-2782 |
| EXON2_EXON3 | 2 | 2878-2882 |
| EXON3 | 3 | 2981-2983 |
| EXON4 | 5 | 3113-3116 |
| EXON5 | 4 | 3397-3401 |
| EXON5_EXON6 | 7 | 3432-3443 |
| EXON6 | 3 | 3464, 3505-3506 |
| EXON6_EXON7 | 11 | 3563-3573 |
| EXON7 | 13 | 3612, 3614-3616, 3618-3619, 3627, 3629, 3631, 3633 |
| EXON8 | 50 | 3749-3759, 3818-3863, 7346-7354, 7394, 8139, 8418-8423, 10358-10363, 10386-10425 |

### Example 2: In vitro cell model activity test of the ASO (HaCaT human immortalized epidermal cells or LNCaP human prostate cancer cells).

In this example, the inhibitory effects of the ASO listed in Table 1 on androgen receptor (AR) expression in HaCaT or LNCaP cells were verified. The experimental procedure is as follows:
(1) Prepare the suspension transfection reagents by dissolving the ASO powder in sterile water to a concentration of 10 µM. Then, dilute the 10 µM ASO stock solution to the desired concentration using UltraFectin^{®} serum-reduced, transfection-specific medium (Basalmedia, L530KJ). Dilute the Lipofectamine 2000 transfection reagent (Invitrogen, 11668-019). Combine the diluted transfection reagent and the diluted ASO solution to create ASO transfection complexes at the desired concentrations or concentration gradients. Pipette up and down ten times to mix thoroughly, then leave to stand at room temperature for twenty minutes..
(2) Cell preparation: Seed the cells one day before transfection. Seed 96-well plates with 6,000 cells per well. Add 100 µL of DMEM medium containing 10% FBS for HaCaT cells, or RPMI 1640 medium containing 10% FBS, 1% sodium pyruvate and 1% glutamine for LNCaP cells, to each well. Observe the HaCaT or LNCaP cells under a microscope prior to transfection; the confluence should be >70%. Aspirate the old medium and replace it with 50 µL of DMEM medium containing 10% FBS for HaCaT cells or RPMI 1640 medium containing 10% FBS for LNCaP cells. Add the ASO transfection complex prepared in step 1 to the 96-well plate. Incubate in a 37 °C, 5% CO₂ incubator and replace the medium with fresh medium after 5 hours.
(3) 24 hours after transfection, total RNA was extracted from the cells and AR mRNA expression was detected using quantitative real-time PCR (qRT-PCR). Table 2 shows the PCR primers and probes used to amplify the housekeeping gene Actin and the target gene AR.

**Table 2: PCR Primer and Probe Sequences**

| Name | Sequence | Nucleotide sequence(5'-3') |
|---|---|---|
| *hAR* | Forward primer | SEQ ID NO :66 GCCCCTGGATGGATAGCTACT |
| | Reverse primer | SEQ ID NO :67 CCACAGATCAGGCAGGTCTTC |
| | Probe | SEQ ID NO :68 ACTGCCAGGGACCATGTTTTGCCC |
| *hActin* | Forward primer | SEQ ID NO :69 GACTACCTCATGAAGATCCTCACC |
| | Reverse primer | SEQ ID NO :70 TCTCCTTAATGTCACGCACGATT |
| | Probe | SEQ ID NO :71 CGGCTACAGCTTCACCACCACGGC |

Relative gene expression was calculated using the 2^(-ΔΔCT) method (also known as the Livak method). The inhibition rate of mRNA expression by antisense oligonucleotides was calculated using the following equation: Inhibition rate = (1 - 2^(-ΔΔCT)) × 100%.

The experimental groups were as follows: Cells treated with the modified ASO, indicated by their respective numbers, and the blank control group (Blank), consisting of cells not treated with any ASO.

The efficiency of AR mRNA inhibition by some of the ASO sequences listed in Table 1 in HaCaT or LNCaP cells is shown in Tables 3-1 to 3-4 below.

The results demonstrate that the ASO sequences in Table 1 significantly inhibit AR mRNA transcription.

**Table 3-1: Inhibition Rate (%) of AR mRNA Following Treatment of HaCaT Cells with the Antisense Oligonucleotides**

| ID | SEQ ID NO | 10nM %inhibition | sequence | Motif modification | startPos( +) | Target region |
|---|---|---|---|---|---|---|
| blank | / | 0 | / | / | / | / |
| NC | 64 | 13 | CCTATAGGACTATCCAGGAA | 5-10-5 | / | / |
| PC | 65 | 50 | AAGTTGTAGTAGTCGC | 4-8-4 | 2211 | EXON1 |
| 150 | 1 | 21 | TGGCGTGGTGCGTCCC | 4-8-4 | 134 | 5UTR EXON1 |
| 13 | 2 | 63 | TGGGGCTGGCGTGGTGCGTCCC | 5-12-5 | 134 | 5UTR EXON1 |
| 161 | 3 | 31 | CTGGCGTGGTGCGTCC | 4-8-4 | 135 | 5UTR EXON1 |
| 134 | 4 | 48 | GCTGGCGTGGTGCGTCC | 4-9-4 | 135 | 5UTR EXON1 |
| 162 | 5 | 34 | GCTGGCGTGGTGCGTC | 4-8-4 | 136 | 5UTR EXON1 |
| 157 | 6 | 10 | GGCTGGCGTGGTGCGT | 4-8-4 | 137 | 5UTR EXON1 |
| 18 | 7 | 60 | GGCTGGGGCTGGCGTGGTGCGT | 5-12-5 | 137 | 5UTR EXON1 |
| 169 | 8 | 20 | GGGCTGGCGTGGTGCG | 4-8-4 | 138 | 5UTR EXON1 |
| 178 | 9 | 10 | GGGGCTGGCGTGGTGC | 4-8-4 | 139 | 5UTR EXON1 |
| 180 | 10 | 33 | CTGGGGCTGGCGTGGT | 4-8-4 | 141 | 5UTR EXON1 |
| 106 | 11 | 59 | GGCTGGGGCTGGCGTGGT | 5-8-5 | 141 | 5UTR EXON1 |
| 182 | 12 | 44 | GCTGGGGCTGGCGTGG | 4-8-4 | 142 | 5UTR EXON1 |
| 181 | 13 | 43 | GGCTGGGGCTGGCGTG | 4-8-4 | 143 | 5UTR EXON1 |
| 183 | 14 | 17 | GGGCTGGGGCTGGCGT | 4-8-4 | 144 | 5UTR EXON1 |
| 153 | 15 | 15 | CGTTGGCTGTCGCTGG | 4-8-4 | 164 | 5UTR EXON1 |
| 89 | 16 | 51 | GGCGTTGGCTGTCGCTGG | 5-8-5 | 164 | 5UTR EXON1 |
| 131 | 17 | 61 | GCGTTGGCTGTCGCTGG | 4-9-4 | 164 | 5UTR EXON1 |
| 164 | 18 | 51 | GCGTTGGCTGTCGCTG | 4-8-4 | 165 | 5UTR EXON1 |
| 120 | 19 | 66 | GGCGTTGGCTGTCGCTG | 4-9-4 | 165 | 5UTR EXON1 |
| 111 | 20 | 65 | AGGCGTTGGCTGTCGCT | 4-9-4 | 166 | 5UTR EXON1 |
| 151 | 21 | 27 | AGGCGTTGGCTGTCGC | 4-8-4 | 167 | 5UTR EXON1 |
| 163 | 22 | 11 | GCTTCGAAGCCGCCGC | 4-8-4 | 191 | 5UTR EXON1 |
| 156 | 23 | -19 | GGCTTCGAAGCCGCCG | 4-8-4 | 192 | 5UTR EXON1 |
| 155 | 24 | -4 | CGGCTTCGAAGCCGCC | 4-8-4 | 193 | 5UTR EXON1 |
| 152 | 25 | 3 | GGGCGGCGGCTTCGAA | 4-8-4 | 199 | 5UTR EXON1 |
| 176 | 26 | 22 | AGGAAAGGGCAGCTCC | 4-8-4 | 215 | 5UTR EXON1 |
| 177 | 27 | 12 | GAGGAAAGGGCAGCTC | 4-8-4 | 216 | 5UTR EXON1 |
| 174 | 28 | -5 | CGAAGAGGAAAGGGCA | 4-8-4 | 220 | 5UTR EXON1 |
| 167 | 29 | 37 | CCGAAGAGGAAAGGGC | 4-8-4 | 221 | 5UTR EXON1 |
| 166 | 30 | 34 | ACCGAAGAGGAAAGGG | 4-8-4 | 222 | 5UTR EXON1 |
| 173 | 31 | 1 | CACCGAAGAGGAAAGG | 4-8-4 | 223 | 5UTR EXON1 |
| 165 | 32 | 22 | TCACCGAAGAGGAAAG | 4-8-4 | 224 | 5UTR EXON1 |
| 172 | 33 | 12 | TTCACCGAAGAGGAAA | 4-8-4 | 225 | 5UTR EXON1 |
| 94 | 34 | 37 | ACTTCACCGAAGAGGAAA | 5-8-5 | 225 | 5UTR EXON1 |
| 158 | 35 | 33 | CTTCACCGAAGAGGAA | 4-8-4 | 226 | 5UTR EXON1 |
| 159 | 36 | 35 | ACTTCACCGAAGAGGA | 4-8-4 | 227 | 5UTR EXON1 |
| 81 | 37 | 43 | AAACTTCACCGAAGAGGA | 5-8-5 | 227 | 5UTR EXON1 |
| 154 | 38 | 12 | AACTTCACCGAAGAGG | 4-8-4 | 228 | 5UTR EXON1 |
| 149 | 39 | 8 | AAACTTCACCGAAGAG | 4-8-4 | 229 | 5UTR EXON1 |
| 170 | 40 | 5 | ACTTTCCTTGCTTCCT | 4-8-4 | 267 | 5UTR EXON1 |
| 179 | 41 | 3 | CACTTTCCTTGCTTCC | 4-8-4 | 268 | 5UTR EXON1 |
| 175 | 42 | 20 | GGACAAAGGCAGCCGT | 4-8-4 | 297 | 5UTR EXON1 |
| 91 | 43 | 34 | GAGGACAAAGGCAGCCGT | 5-8-5 | 297 | 5UTR EXON1 |
| 168 | 44 | 41 | AGGACAAAGGCAGCCG | 4-8-4 | 298 | 5UTR EXON1 |
| 237 | 45 | 72 | CGCGCACGCTCTGGAACAGA | 5-10-5 | 1201 | EXON1 |
| 194 | 46 | 70 | GAAAGTTGTAGTAGTCGCGA | 5-10-5 | 2209 | EXON1 |
| 271 | 47 | 82 | AAGTTGTAGTAGTCGCGA | 5-8-5 | 2209 | EXON1 |
| 204 | 48 | 63 | GGAAAGTTGTAGTAGTCGCG | 5-10-5 | 2210 | EXON1 |
| 272 | 49 | 48 | AAAGTTGTAGTAGTCGCG | 5-8-5 | 2210 | EXON1 |
| 250 | 50 | 83 | TGCATGCGGTACTCATTGAA | 5-10-5 | 3437 | EXON5_EXON6 |
| 193 | 51 | 51 | GTGCATGCGGTACTCATTGA | 5-10-5 | 3438 | EXON5_EXON6 |
| 219 | 52 | 78 | TGCCAGTGAACATACATAAA | 5-10-5 | 7346 | EXON8 3UTR |
| 192 | 53 | 69 | GTGCCAGTGAACATACATAA | 5-10-5 | 7347 | EXON8 3UTR |
| 289 | 54 | 77 | TGCCAGTGAACATACATA | 5-8-5 | 7348 | EXON8 3UTR |
| 285 | 55 | 66 | TAGTGCCAGTGAACATAC | 5-8-5 | 7351 | EXON8 3UTR |
| 4 | 56 | 71 | TTTTTAGTGCCAGTGAACAT | 5-10-5 | 7353 | EXON8 3UTR |
| 300 | 57 | 72 | TTTTAGTGCCAGTGAACA | 5-8-5 | 7354 | EXON8 3UTR |
| 206 | 58 | 73 | GAGGCACTAATGCTTGCTCC | 5-10-5 | 8139 | EXON8 3UTR |
| 5 | 59 | 83 | AACAGAACACTAGCGCTTGG | 5-10-5 | 10358 | EXON8 3UTR |
| 3 | 60 | 74 | GAACAGAACACTAGCGCTTG | 5-10-5 | 10359 | EXON8 3UTR |
| 241 | 61 | 83 | AGAGAACAGAACACTAGCGC | 5-10-5 | 10362 | EXON8 3UTR |
| 280 | 62 | 48 | GAGAACAGAACACTAGCG | 5-8-5 | 10363 | EXON8 3UTR |

| | | | | | | |
|---|---|---|---|---|---|---|
| **[0043]** Note: Motif Indicates the MOE modification of the 3' and/or 5' end bases, for example, [0044] 4-8-4 indicates MOE modification of 4 bases at the 3' end and 4 bases at the 5' end; [0045] 5-10-5 indicates MOE modification of 5 bases at the 3' end and 5 bases at the 5' end. | | | | | | |

**Table 3-2: Inhibition Rate (%) of AR mRNA Following Treatment of HaCaT Cells with the Antisense Oligonucleotides**

| ID | Administration concentration | | | | | |
|---|---|---|---|---|---|---|
| | 30nM | 10nM | 3nM | 1nM | 0.3nM | 0.1nM |
| NC | -28 | 4 | 1 | -17 | 21 | 18 |
| PC | 74 | 54 | 23 | 8 | 0 | 22 |
| 3 | 76 | 64 | 34 | 31 | -5 | -1 |
| 5 | 68 | 65 | 45 | 29 | -18 | -3 |
| 194 | 93 | 80 | 64 | 44 | 24 | 5 |
| 204 | 85 | 79 | 57 | 27 | 11 | 17 |
| 250 | 68 | 60 | 40 | 18 | -10 | -5 |
| 289 | 79 | 79 | 69 | 46 | 23 | 23 |

**Table 3-3: Inhibition Rate (%) of AR mRNA Following Treatment of LNCaP Cells with the Antisense Oligonucleotides**

| ID | 10nM %inhibition | ASO | Motif | startPos(+) | Target region |
|---|---|---|---|---|---|
| blank | -5 | / | | / | / |
| NC | 31 | CCTATAGGACTATCCAGGAA | | / | / |
| PC | 65 | AAGTTGTAGTAGTCGC | 4-8-4 | 2211 | EXON1 |
| 0149 | 5 | AAACTTCACCGAAGAG | 4-8-4 | 229 | 5UTR EXON1 |
| 0150 | 40 | TGGCGTGGTGCGTCCC | 4-8-4 | 134 | 5UTR EXON1 |
| 0151 | 34 | AGGCGTTGGCTGTCGC | 4-8-4 | 167 | 5UTR EXON1 |
| 0152 | 6 | GGGCGGCGGCTTCGAA | 4-8-4 | 199 | 5UTR EXON1 |
| 0153 | 3 | CGTTGGCTGTCGCTGG | 4-8-4 | 164 | 5UTR EXON1 |
| 0154 | 6 | AACTTCACCGAAGAGG | 4-8-4 | 228 | 5UTR EXON1 |
| 0155 | 28 | CGGCTTCGAAGCCGCC | 4-8-4 | 193 | 5UTR EXON1 |
| 0156 | 11 | GGCTTCGAAGCCGCCG | 4-8-4 | 192 | 5UTR EXON1 |
| 0157 | 40 | GGCTGGCGTGGTGCGT | 4-8-4 | 137 | 5UTR EXON1 |
| 0158 | 35 | CTTCACCGAAGAGGAA | 4-8-4 | 226 | 5UTR EXON1 |
| 0159 | 1 | ACTTCACCGAAGAGGA | 4-8-4 | 227 | 5UTR EXON1 |
| 0161 | 39 | CTGGCGTGGTGCGTCC | 4-8-4 | 135 | 5UTR EXON1 |
| 0162 | 49 | GCTGGCGTGGTGCGTC | 4-8-4 | 136 | 5UTR EXON1 |
| 0163 | 60 | GCTTCGAAGCCGCCGC | 4-8-4 | 191 | 5UTR EXON1 |
| 0164 | 38 | GCGTTGGCTGTCGCTG | 4-8-4 | 165 | 5UTR EXON1 |
| 0165 | 18 | TCACCGAAGAGGAAAG | 4-8-4 | 224 | 5UTR EXON1 |
| 0166 | 46 | ACCGAAGAGGAAAGGG | 4-8-4 | 222 | 5UTR EXON1 |
| 0167 | 35 | CCGAAGAGGAAAGGGC | 4-8-4 | 221 | 5UTR EXON1 |
| 0168 | 32 | AGGACAAAGGCAGCCG | 4-8-4 | 298 | 5UTR EXON1 |
| 0169 | 47 | GGGCTGGCGTGGTGCG | 4-8-4 | 138 | 5UTR EXON1 |
| 0170 | 42 | ACTTTCCTTGCTTCCT | 4-8-4 | 267 | 5UTR EXON1 |
| 0172 | 32 | TTCACCGAAGAGGAAA | 4-8-4 | 225 | 5UTR EXON1 |
| 0173 | 48 | CACCGAAGAGGAAAGG | 4-8-4 | 223 | 5UTR EXON1 |
| 0174 | 36 | CGAAGAGGAAAGGGCA | 4-8-4 | 220 | 5UTR EXON1 |
| 0175 | 62 | GGACAAAGGCAGCCGT | 4-8-4 | 297 | 5UTR EXON1 |
| 0176 | 60 | AGGAAAGGGCAGCTCC | 4-8-4 | 215 | 5UTR EXON1 |
| 0177 | 64 | GAGGAAAGGGCAGCTC | 4-8-4 | 216 | 5UTR EXON1 |
| 0178 | 41 | GGGGCTGGCGTGGTGC | 4-8-4 | 139 | 5UTR EXON1 |
| 0179 | 7 | CACTTTCCTTGCTTCC | 4-8-4 | 268 | 5UTR EXON1 |
| 0180 | 7 | CTGGGGCTGGCGTGGT | 4-8-4 | 141 | 5UTR EXON1 |
| 0181 | 44 | GGCTGGGGCTGGCGTG | 4-8-4 | 143 | 5UTR EXON1 |
| 0182 | 38 | GCTGGGGCTGGCGTGG | 4-8-4 | 142 | 5UTR EXON1 |
| 0183 | 35 | GGGCTGGGGCTGGCGT | 4-8-4 | 144 | 5UTR EXON1 |

**Table 3-4: Inhibition Rate (%) of AR mRNA Following Treatment of LNCaP Cells with Antisense Oligonucleotides**

| | Administration concentration | | | | | |
|---|---|---|---|---|---|---|
| ID | 30nM | 10nM | 3nM | 1nM | 0.3nM | 0.1nM |
| NC | 12 | -18 | -5 | 14 | -1 | -3 |
| PC | 80 | 76 | 24 | -5 | -5 | 5 |
| 3 | 78 | 76 | 47 | 35 | 23 | 43 |
| 5 | 75 | 62 | 52 | 30 | 3 | -19 |
| 194 | 85 | 70 | 34 | 24 | 2 | -5 |
| 204 | 65 | 83 | 42 | 66 | 65 | 69 |
| 250 | 84 | 66 | 46 | 22 | -3 | 14 |
| 289 | 88 | 87 | 79 | 74 | 30 | 27 |

### Example 3: Testing the activity of ASO using an in vitro cell model (LNCaP human prostate cancer cells)

In this example, the inhibitory effect of the ASO molecules listed in Table 1 on AR protein levels in LNCaP cells was verified. The transfection reagent was prepared as described in Example 2. The experimental procedure is as follows:
(1) A 6-well plate was used with a cell density of 1.5 × 10⁵ cells per well;
(2) The concentration of ASO used to treat the cells was 10nM.
(3) 72 hours after ASO treatment, total protein was extracted from the treated LNCaP cells using RIPA lysis buffer (Beyotime, P0013B), supplemented with PMSF (Beyotime, ST505) and protease inhibitors.
(4) The proteins were separated using sodium dodecyl sulfate polyacrylamide gel electrophoresis (SDS-PAGE) and transferred onto a PVDF membrane. After blocking with 5% non-fat dry milk, the membrane was incubated overnight at 4 °C with anti-AR antibody (Abcam, ab133273) and anti-actin antibody (Abcam, ab49900). The following day, the membrane was incubated with the secondary antibody (CST, 7074S) at room temperature for one hour. Protein expression levels were visualised using a multi-mode imaging system (Tanon 5200 Multi).

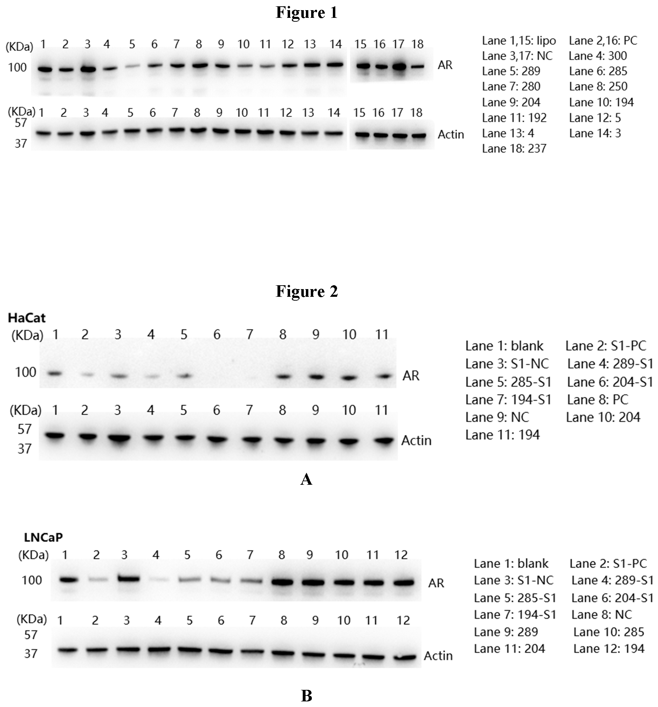

Figure 1 and Table 4 (partial results only) show the effects of the ASO listed in Table 1 on inhibiting AR protein expression in LNCaP human prostate cancer cells.

The results indicate that the ASO shown in Table 1 significantly inhibit AR protein expression level in LNCaP cells.

**Table 4: Inhibition rate (%) of AR protein in LNCaP cells by ASO (WB experiment)**

| ID. | Inhibition rate (%) |
|---|---|
| PC | 26 |
| NC | -9 |
| 300 | 48 |
| 289 | 87 |
| 285 | 55 |
| 280 | 40 |
| 250 | 26 |
| 204 | 46 |
| 194 | 67 |
| 192 | 74 |
| 5 | 45 |
| 4 | 15 |
| 3 | 12 |
| 237 | 59 |

### Example 4: Testing the activity of the antisense oligonucleotide-fatty acid construct (ASO-S1) in vitro (cell models, HaCaT human immortalized epidermal cells and NCaP human prostate cancer cells)

In this example, the inhibitory effect of the ASO-S1 construct, which is derived from the ASO molecule shown in Table 1 and has been modified with a fatty acid chain, on the expression of the androgen receptor (AR) in HaCaT/LNCaP cells was verified. The specific experimental procedure is as follows:
(1) Preparation of the incubation medium: Prepare DMEM (for HaCaT cells) or RPMI 1640 (for LNCaP cells) containing 1% foetal bovine serum (FBS) and 1% insulin-transferrin-selenium-aminoethanol (GIBCO, 51500056). Dissolve the ASO-S1 powder in sterile water to reach a concentration of 1 mM. (2) Cell treatment: dilute the 1 mM ASO-S1 stock solution with the prepared medium to create ASO-S1 incubation complexes at predetermined concentrations or concentration gradients. Mix by pipetting up and down 10 times.
(2) Cell treatment: Seed the cells one day prior to incubation. Seed 6-well plates at a density of 1.5 × 10⁵ cells per well. Add 2,000 µL of DMEM medium containing 10% FBS for HaCaT cells or RPMI 1640 medium containing 10% FBS, 1% sodium pyruvate and 1% glutamine for LNCaP cells. Prior to transfection, observe the HaCaT and LNCaP cells under a microscope; the confluence should be greater than 70%. Discard the old medium and add 2,000 µL of the incubation reagent prepared in step 1 to each well. Incubate at 37 °C in 5% CO₂ for 24 hours before replacing with complete medium.
(3)After 72 hours of incubation, total cellular protein was extracted and subsequent steps were performed as described in Example 3.

The efficiency of the ASO-S1 construct in inhibiting AR protein in HaCaT/LNCaP cells, based on the ASO sequence shown in Table 1, is shown in Table 5 and Figure 2 (partial results only).

The results demonstrate that the ASO-S1 construct shown in Table 1 exhibits a notable inhibitory effect on AR protein at an incubation concentration of 10 µM.

**Table 5: Inhibition rates (%), HaCaT/LNCaP cells, AR protein, ASO-S1 construct**

| ID | Inhibition rates(HaCaT 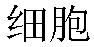 ) | Inhibition rates(LNCaP 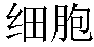 ) |
|---|---|---|
| S1-PC | 74 | 82 |
| S1-NC | 36 | 20 |
| 289-S1 | 69 | 95 |
| 285-S1 | 21 | 76 |
| 204-S1 | 92 | 75 |
| 194-S1 | 84 | 55 |
| PC | -50 | NA |
| NC | -64 | -8 |
| 289 | NA | -17 |
| 285 | NA | -4 |
| 204 | -62 | 12 |
| 194 | -42 | -1 |

### Example 6: serum stability of the antisense oligonucleotides (ASO) in fetal bovine serum and rat serum.

In this example, it was confirmed that the ASO molecules shown in Table 1 remained stable in serum for at least 48 hours.
(1) Serum solutions with concentrations of 10% and 50% were prepared using sterile water. A mass of 0.3 µg of ASO solution was added to 50 µL of each serum concentration, mixed thoroughly and then incubated at 37 °C for 0, 4 and 24 hours, respectively.
(2) After incubation at all time points, DNA was extracted using DNA extraction buffer (Solarbio, P1021). The DNA was then precipitated using a mixture of 100% ethanol, 3 M sodium acetate and glycogen, before being dissolved in ddH₂O.
(3) The DNA was separated using 15% TBE-UREA polyacrylamide gel electrophoresis (PAGE), stained with a safe nucleic acid dye (Monad, ME20301) and the quantity of DNA observed using a multifunctional imaging system (Tanon 5200 Multi).

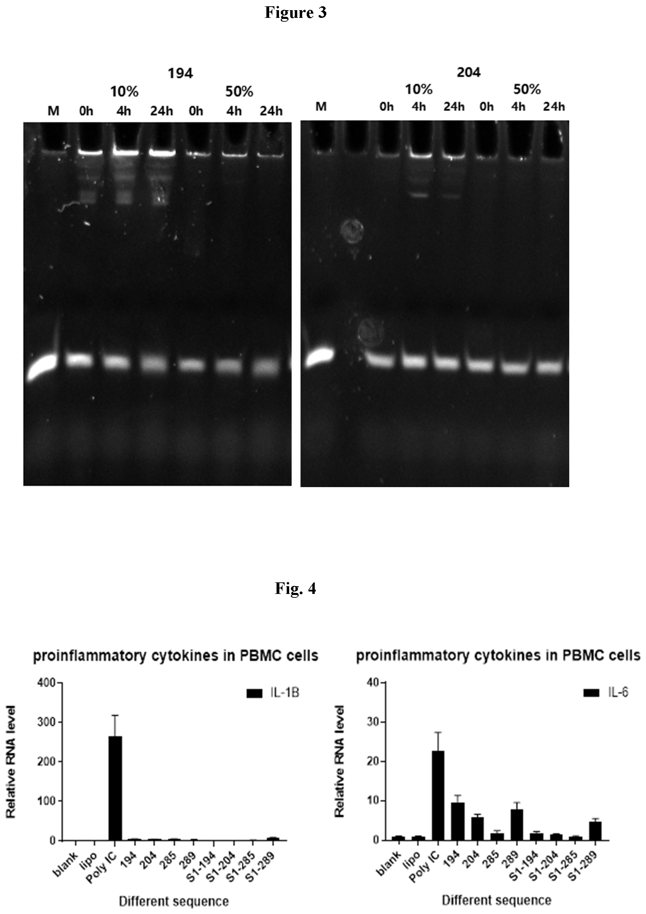

Figure 3 shows the stability of some of the ASO listed in Table 1 and their corresponding ASO-S1 constructs in serum.

The results indicate that the ASO listed in Table 1 and their corresponding ASO-S1 constructs remain stable in serum for at least 24 hours.

### Example 7: Detection of the cytokines associated with inflammatory responses in an in vitro cell model (peripheral blood mononuclear cells, or PBMCs) after treatment with the ASO and ASO-S1 construct.

In this example, it was demonstrated that PBMC cells treated with some of the antisense oligonucleotide (ASO)/antisense oligonucleotide-S1 (ASO-S1) compounds listed in Table 1 exhibited significantly lower levels of cytokines associated with inflammatory responses than cells treated with the positive control. The specific experimental procedure is as follows:
(1) Cell treatment: Cell thawing and seeding were performed one day prior to incubation. The cells were seeded at a density of 1 × 10⁶ cells per well in a 12-well plate. To each well, 100 µL of RPMI 1640 medium containing 1% FBS and 1% insulin-transferrin-selenium-aminoethanol (GIBCO, 51500056) was added.
(2) Incubation: The appropriate volume of ASO/ASO-S1 was added directly to each well from step (1) to achieve a final concentration of 10 µM. Alternatively, the appropriate volume of positive control Poly IC (Sigma, P9582) was added to each well to achieve a final concentration of 2 µg/mL. Mix well and incubate in a 37 °C, 5% CO₂ incubator.
(3) After 24 hours, total RNA was extracted from the cells and cytokine mRNA expression was detected using quantitative real-time PCR (qRT-PCR). The PCR primers used to amplify the internal reference gene RPL13A and the target genes IL-6 and IL-1β are shown in Table 6.

**Table 6: PCR primer and probe sequences for cytokines**

| cytokine | primer | sequence(5'-3') |
|---|---|---|
| *IL-1β* | Forward primer | SEQ ID NO :72 CTGAGCTCGCCAGTGAAAT |
| | Reverse primer | SEQ ID NO :73 CTGTAGTGGTGGTCGGAGA |
| *IL-6* | Forward primer | SEQ ID NO :74 AGATGCAATAACCACCCCTG |
| | Reverse primer | SEQ ID NO :75 TGCGCAGAATGAGATGAGTT |
| RPL13A | Forward primer | SEQ ID NO :76 TGCCATTGCCCCATGTGAAG |
| | Reverse primer | SEQ ID NO :77 AGCTGCACATCACTCAGGATT |

The results are shown in Figure 4: After treatment with the ASO/ASO-S1, the effect on inflammatory factors were lower.

Example 8: Testing the antitumor activity of the ASO-S1 (LNCaP cell, human prostate cancer cells) in vitro.

In this example, it was demonstrated that the ASO-S1 construct derived from the ASO molecule (modification of the fatty acid chain) shown in Table 1 exhibits a certain cytotoxic effect on LNCaP prostate cancer cells. The specific experimental procedure is as follows:
(1) Preparation of incubation reagents: RPMI 1640 medium containing 1% FBS and 1% insulin-transferrin-selenium-aminoethanol (GIBCO, 51500056) was prepared. ASO-S1 powder was dissolved in sterile water to a concentration of 1 mM. (2) Cell treatment: dilute the 1 mM ASO-S1 stock solution with the prepared medium to create ASO-S1 incubation complexes at varying concentrations. Mix by pipetting up and down 10 times.
(2) Cell treatment: Cell seeding was performed one day prior to incubation. A 96-well plate was seeded with 10,000 cells per well and 100 µL of RPMI 1640 medium containing 10% FBS, 1% sodium pyruvate and 1% glutamine was added to each well. Observe the condition of the LNCaP cells under a microscope prior to treatment. Discard the old medium and add 100 µL of the incubation reagent prepared in step 1 to each well. Incubate in a 37 °C, 5% CO₂ incubator.
(3) After 72 hours, perform the CCK-8 assay.

**Table 7: Growth inhibition rate of ASO-S1 against LNCaP cells (%)**

| ID | Administration concentration | | | | | |
|---|---|---|---|---|---|---|
| | 10µM | 3µM | 1µM | 0.3µM | 0.1µM | 0.03µM |
| S1-NC | 10 | 4 | 1 | -1 | 3 | 3 |
| S1-PC | 57 | 40 | 7 | 9 | 2 | 3 |
| 204-S1 | 46 | 33 | 13 | 0 | -11 | 6 |
| 250-S1 | 62 | 41 | 11 | 12 | 15 | 4 |
| 289-S1 | 24 | 36 | 17 | 5 | 4 | -4 |
| 003-S1 | 37 | 19 | -9 | -2 | -1 | 2 |

As shown in Table 7, the tested ASO-S1 also inhibited the growth of prostate cancer cells after treatment.

### Example 9: Preparation of a topical formulation

1. Prepare a topical formulation containing ASO/ASO-S1, following the prescription in Table 8 below.

**Table 8: Topical formulation containing ASO/ASO-S1**

| Substance | Concentration(mg/mL) |
|---|---|
| ASO or ASO-S1(API) | 5-50 |
| SNAC | 5~50(preferably 30) |
| DMI | 5~60(preferably 40) |
| TPGS | 2~20(preferably 10) |
| Vitamin C | 2~20(preferably 10) |

Sodium 8-(2-hydroxybenzoylamido)octanoate (SNAC)
Dimethyl isosorbide anhydride (DMI)
Vitamin E polyethylene glycol succinate (TPGS)
ASO or ASO-S1 is selected from the ASO or its chemically modified forms described in the aforementioned embodiments.

2. The method for preparing a topical formulation containing ASO/ASO-S1 involves the following steps:
2.1. Dissolved oxygen equilibration: take purified water (prepared using a water purifier), insert a nitrogen tube below the liquid surface and open the main nitrogen valve. Slowly adjust the nitrogen flow from the valve to reach a pressure of approximately 0.1-0.2 MPa. Repeat this operation for subsequent nitrogen filling. The nitrogen filling time is 6 hours to allow the dissolved oxygen in the water to equilibrate.
2.2. Dissolution: Add the required amounts of SNAC, DMI, vitamin C and TPGS to the appropriate volume of oxygen-equilibrated water. Stir until everything has dissolved.
2.3. pH adjustment
   Adjust the pH to between 7.5 and 9.0, then nitrogen flush for two hours to re-equilibrate the dissolved oxygen.
2.4. API dissolution
   Add the API (ASO or ASO-S1) to the solution obtained in step 2.4 and dissolve it.
2.5. Volumetric adjustment
   Adjust the volume with oxygen-equilibrated water. After volumetric adjustment, purge with nitrogen for two hours to re-equilibrate the dissolved oxygen, thereby preparing the ASO/ASO-S1 topical formulation.
2.6. Filling and labelling
   Transfer the formulation to borosilicate glass vials. After purging with nitrogen, seal with a rubber stopper and aluminum cap, then cap and label.

Example 10: Evaluation of hair regrowth function of the topical formulations.

In this example, the hair regrowth function of three topical formulations containing different ASO-S1, prepared according to the method of Example 9 , were evaluated.

This investigator-initiated clinical trial (IIT) was conducted at West China Hospital, Sichuan University.
1. Ethics approval number: Approval from the Biomedical Ethics Review Committee of West China Hospital, Sichuan University (No. 2024-80).
2. Product usage: Male subjects clinically diagnosed with androgenetic alopecia (aged 18-60 years, Hamilton-Norwood grades IIIa, III, IIIv, IV, IVa, or V) were enrolled in this IIT study.

The product is for topical application. The single dose is 1 ml (1 applicator). Treatment begins with a once-daily application for three consecutive days, followed by a once-weekly application. The product should be used at the same time each week for a continuous treatment duration of no less than three months. For patients with severe hair loss, the single dose may be increased to 2 ml (two applicators), based on professional advice, with the same frequency of use as above. The placebo group received a blank excipient solution containing no API.

The product is a topical formulation that is used with a massage applicator. The product must be applied immediately after washing and blow-drying the hair with warm air. Pour the product into the applicator and apply it evenly to the balding areas of the scalp, massaging for 3-5 minutes. Allow the scalp to dry naturally after application and avoid washing the hair for 24 hours.

Hair density in the application area was measured before and after use. The hair regrowth effects for each treatment group are shown in Tables 9-1 to 9-7.

**Table 9-1: Statistical summary of before and after taking the medication in the placebo group**

| **No.** | **ID** | **Gender** | **Age** | **Baseline hair density (Hairs/cm²)** | **Hair density at Week 12 (Hairs/cm²)** |
|---|---|---|---|---|---|
| 1 | 1 | male | 40 | 185.5 | 183.1 |
| 2 | 8 | male | 37 | 166.1 | 162.7 |
| 3 | 11 | male | 55 | 110.5 | 110.5 |
| 4 | 12 | male | 49 | 196.6 | 177.0 |
| 5 | 13 | male | 35 | 192.6 | 198.2 |
| 6 | 14 | male | 40 | 187.8 | 203.2 |
| 7 | 16 | male | 33 | 146.5 | 149.7 |
| 8 | 19 | male | 34 | 215.0 | 188.5 |
| 9 | 20 | male | 28 | 239.3 | 233.1 |
| 10 | 21 | male | 27 | 242.5 | 233.3 |
| 11 | 25 | male | 40 | 239.1 | 235.1 |
| 12 | 27 | male | 27 | 191.9 | 166.4 |
| 13 | 30 | male | 45 | 161.0 | 150.7 |
| 14 | 31 | male | 24 | 231.5 | 215.0 |
| 15 | 32 | male | 35 | 187.5 | 170.6 |
| 16 | 35 | male | 52 | 198.0 | 191.0 |
| 17 | 40 | male | 30 | 200.9 | 195.6 |
| 18 | 41 | male | 34 | 224.8 | 231.3 |
| 19 | 42 | male | 52 | 70.5 | 92.7 |
| 20 | 43 | male | 33 | 128.9 | 122.9 |
| 21 | 44 | male | 42 | 77.3 | 82.4 |
| 22 | 45 | male | 40 | 174.5 | 166.4 |
| 23 | 50 | male | 34 | 292.1 | 308.1 |
| 24 | 54 | male | 31 | 201.3 | 204.5 |
| 25 | 64 | male | 36 | 219.7 | 187.8 |
| 26 | 66 | male | 32 | 210.8 | 209.7 |
| 27 | 68 | male | 50 | 189.7 | 187.4 |
| **Average** | | | **37.6** | **188.2** | **183.6** |

The average increase in hair density in the placebo group was: -2.44%,
calculated as: (183.6 - 188.2) / 188.2 × 100% (same formula applies below).
30% of patients in the placebo group, hair density did not decrease.

**Table 9-2: Statistical Summary of before and after taking the medication in the Formulation (4 mg/mL LT010-0390) Group**

| **No.** | **ID** | **Gender** | **Age** | **Baseline hair density (Hairs/cm²)** | **Hair density at Week 12 (Hairs/cm²)** |
|---|---|---|---|---|---|
| 1 | 5 | male | 51 | 185.9 | 167.8 |
| 2 | 6 | male | 37 | 172.4 | 190.3 |
| 3 | 7 | male | 30 | 201.2 | 204.3 |
| 4 | 9 | male | 58 | 171.1 | 169.1 |
| 5 | 10 | male | 52 | 161.9 | 160.8 |
| 6 | 15 | male | 36 | 126.1 | 131.3 |
| 7 | 17 | male | 34 | 162.9 | 177.0 |
| 8 | 22 | male | 34 | 198.8 | 191.9 |
| 9 | 23 | male | 35 | 142.2 | 146.5 |
| 10 | 24 | male | 35 | 197.0 | 186.6 |
| 11 | 26 | male | 41 | 193.9 | 201.6 |
| 12 | 28 | male | 37 | 180.2 | 167.2 |
| 13 | 33 | male | 46 | 135.6 | 116.5 |
| 14 | 34 | male | 36 | 241.7 | 258.1 |
| **Average** | | | **40.1** | **176.5** | **176.3** |

The average increase in hair density in this treatment group was: -0.11%. This compares to an average increase of 2.33% in the placebo group.

Hair density increased in 50% of patients; with an average increase of 6.53% among these responders.

**Table 9-3: Statistical Summary of before and after taking the medication in the Formulation (10 mg/mL LT010-0390) Group**

| **No.** | **ID** | **Gender** | **Age** | **Baseline hair density (Hairs/cm²)** | **Hair density at Week 12 (Hairs/cm²)** |
|---|---|---|---|---|---|
| 1 | 38 | male | 47 | 175.4 | 179.6 |
| 2 | 39 | male | 42 | 204.4 | 199.4 |
| 3 | 46 | male | 41 | 217.4 | 227.1 |
| 4 | 48 | male | 40 | 155.5 | 167.6 |
| 5 | 49 | male | 43 | 177.7 | 163.4 |
| 6 | 52 | male | 33 | 203.9 | 201.8 |
| 7 | 53 | male | 24 | 226.6 | 232.0 |
| 8 | 58 | male | 33 | 189.0 | 187.9 |
| 9 | 59 | male | 30 | 219.7 | 215.3 |
| 10 | 60 | male | 48 | 175.0 | 198.7 |
| 11 | 61 | male | 31 | 198.2 | 190.9 |
| 12 | 62 | male | 35 | 216.9 | 214.9 |
| 13 | 63 | male | 36 | 164.7 | 176.8 |
| 14 | 65 | male | 39 | 123.4 | 120.3 |
| 15 | 67 | male | 37 | 151.0 | 173.2 |
| **Average** | | | **37.3** | **186.6** | **189.9** |

The average increase in hair density in this treatment group was: 1.77%. This compares to an average increase of 4.21% in the placebo group.

Hair density increased in 46% of patients; with an average increase of 7.06% among these responders.

**Table 9-4: Statistical Summary of before and after taking the medication in the Formulation (4 mg/mL LT010-0410) Group**

| **No.** | **ID** | **Gender** | **Age** | **Baseline hair density(Hairs/cm²)** | **Hair density at Week 12(Hairs/cm²)** |
|---|---|---|---|---|---|
| 1 | 104 | male | 35 | 213.3 | 235.1 |
| 2 | 107 | male | 46 | 124.0 | 121.0 |
| 3 | 108 | male | 42 | 135.4 | 154.3 |
| 4 | 109 | male | 37 | 181.1 | 183.0 |
| 5 | 111 | male | 35 | 210.8 | 211.3 |
| 6 | 113 | male | 42 | 196.9 | 203.4 |
| 7 | 115 | male | 38 | 196.4 | 201.9 |
| 8 | 117 | male | 31 | 114.1 | 124.0 |
| 9 | 121 | male | 33 | 131.0 | 143.4 |
| 10 | 122 | male | 42 | 107.6 | 119.0 |
| 11 | 123 | male | 38 | 140.9 | 138.9 |
| 12 | 125 | male | 39 | 149.8 | 144.3 |
| 13 | 126 | male | 35 | 132.9 | 126.5 |
| 14 | 129 | male | 40 | 156.7 | 163.2 |
| 15 | 130 | male | 34 | 225.7 | 216.8 |
| 16 | 132 | male | 36 | 219.2 | 221.2 |
| 17 | 135 | male | 40 | 171.1 | 172.6 |
| **Average** | | | **37.8** | **165.1** | **169.4** |

The average increase in hair density in this treatment group was: 2.60%. This compares to an average increase of 5.04% in the placebo group.

Hair density increased in 65% of patients; with an average increase of 5.34% among these responders.

**Table 9-5: Statistical Summary of before and after taking the medication in the Formulation (10 mg/mL LT010-0410) Group**

| **No.** | **ID** | **Gender** | **Age** | **Baseline hair density (Hairs/cm²)** | **Hair density at Week 12 (Hairs/cm²)** |
|---|---|---|---|---|---|
| 1 | 72 | male | 49 | 189.0 | 187.5 |
| 2 | 74 | male | 49 | 220.2 | 231.6 |
| 3 | 75 | male | 33 | 136.9 | 148.3 |
| 4 | 76 | male | 29 | 174.1 | 181.1 |
| 5 | 77 | male | 35 | 153.3 | 168.7 |
| 6 | 79 | male | 36 | 119.0 | 117.6 |
| 7 | 82 | male | 37 | 142.4 | 164.2 |
| 8 | 83 | male | 40 | 133.9 | 136.4 |
| 9 | 86 | male | 31 | 204.9 | 212.8 |
| 10 | 91 | male | 39 | 128.5 | 120.5 |
| 11 | 94 | male | 27 | 154.3 | 172.1 |
| 12 | 97 | male | 31 | 154.8 | 173.6 |
| 13 | 98 | male | 22 | 164.7 | 154.3 |
| 14 | 99 | male | 32 | 184.0 | 176.6 |
| 15 | 100 | male | 46 | 199.9 | 211.3 |
| 16 | 101 | male | 46 | 130.5 | 128.5 |
| **Average** | | | **36.4** | **161.9** | **167.8** |

The average increase in hair density in this treatment group was: 3.64%. This compares to an average increase of 6.09% in the placebo group.

Hair density increased in 62.5% of patients; with an average increase of 7.49% among these responders.

**Table 9-6: Statistical Summary of before and after taking the medication in the Formulation (4 mg/mL LT010-0449) Group**

| **No.** | **ID** | **Gender** | **Age** | **Baseline hair density (Hairs/cm²)** | **Hair density at Week 12 (Hairs/cm²)** |
|---|---|---|---|---|---|
| 1 | 103 | male | 44 | 237.6 | 244.0 |
| 2 | 105 | male | 34 | 207.8 | 218.8 |
| 3 | 106 | male | 26 | 106.6 | 105.7 |
| 4 | 110 | male | 44 | 107.6 | 120.5 |
| 5 | 112 | male | 41 | 124.0 | 131.9 |
| 6 | 114 | male | 35 | 205.4 | 216.8 |
| 7 | 116 | male | 29 | 219.2 | 212.8 |
| 8 | 118 | male | 39 | 188.5 | 202.4 |
| 9 | 119 | male | 32 | 176.6 | 168.7 |
| 10 | 120 | male | 36 | 182.0 | 218.3 |
| 11 | 124 | male | 34 | 223.7 | 223.7 |
| 12 | 127 | male | 22 | 133.4 | 115.1 |
| 13 | 128 | male | 38 | 227.2 | 217.3 |
| 14 | 131 | male | 52 | 131.4 | 133.9 |
| 15 | 133 | male | 30 | 206.3 | 234.6 |
| 16 | 134 | male | 34 | 168.2 | 176.6 |
| 17 | 136 | male | 37 | 120.5 | 128.0 |
| **Average** | | | **35.7** | **174.5** | **180.5** |

The average increase in hair density in this treatment group was: 3.44%. This compares to an average increase of 5.88% in the placebo group.

Hair density increased in 65% of patients; with an average increase of 7.80% among these responders.

**Table 9-7: Statistical Summary of before and after taking the medication in the Formulation (10 mg/mL LT010-0449) Group**

| **No.** | **ID** | **Gender** | **Age** | **Baseline hair density (Hairs/cm²)** | **Hair density at Week 12 (Hairs/cm²)** |
|---|---|---|---|---|---|
| 1 | 69 | male | 47 | 171.6 | 173.1 |
| 2 | 70 | male | 42 | 161.2 | 186.5 |
| 3 | 71 | male | 37 | 105.2 | 113.1 |
| 4 | 73 | male | 37 | 135.4 | 154.3 |
| 5 | 78 | male | 45 | 205.4 | 187.0 |
| 6 | 80 | male | 49 | 134.4 | 144.3 |
| 7 | 81 | male | 39 | 133.9 | 143.4 |
| 8 | 84 | male | 39 | 169.1 | 142.9 |
| 9 | 85 | male | 41 | 129.5 | 116.6 |
| 10 | 87 | male | 35 | 185.0 | 214.3 |
| 11 | 88 | male | 47 | 214.8 | 207.8 |
| 12 | 89 | male | 31 | 164.7 | 154.3 |
| 13 | 92 | male | 41 | 154.8 | 162.7 |
| 14 | 93 | male | 37 | 212.3 | 225.2 |
| 15 | 95 | male | 31 | 176.1 | 184.5 |
| 16 | 96 | male | 31 | 200.4 | 200.9 |
| 17 | 102 | male | 57 | 197.9 | 193.9 |
| **Average** | | | **40.4** | **167.7** | **170.9** |

The average increase in hair density in this treatment group was: 1.91%. This compares to an average increase of 4.35% in the placebo group.

Hair density increased in 65% of patients; with an average increase of 8.38% among these responders.

The API structures (ASO-S1 construct) used in the three formulations in this study are as follows:
(1) LT-0390 (ASO ID. 289):
   T*G*C*C*A*g*t*g*a*a*c*a*t*A*C*A*T*A*HSA,
   modified as follows:
   *: phosphorothioate inter-nucleoside linkage;
   A, G, C, T: 2'-O-methoxyethyl; (2'-MOE);
   a, g, c, t: 2'-deoxy;
   all C base or c base is 5-methyl-modified cytosine;
   HSA: 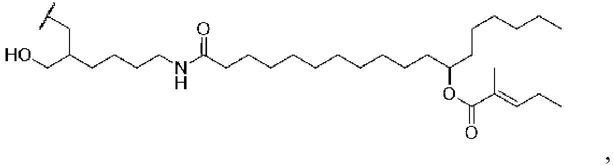
   The APIs can be a hydrogen form (H), a sodium salt form (Na), or S⁻ form, with structures as shown below:
      LT-0390 H : 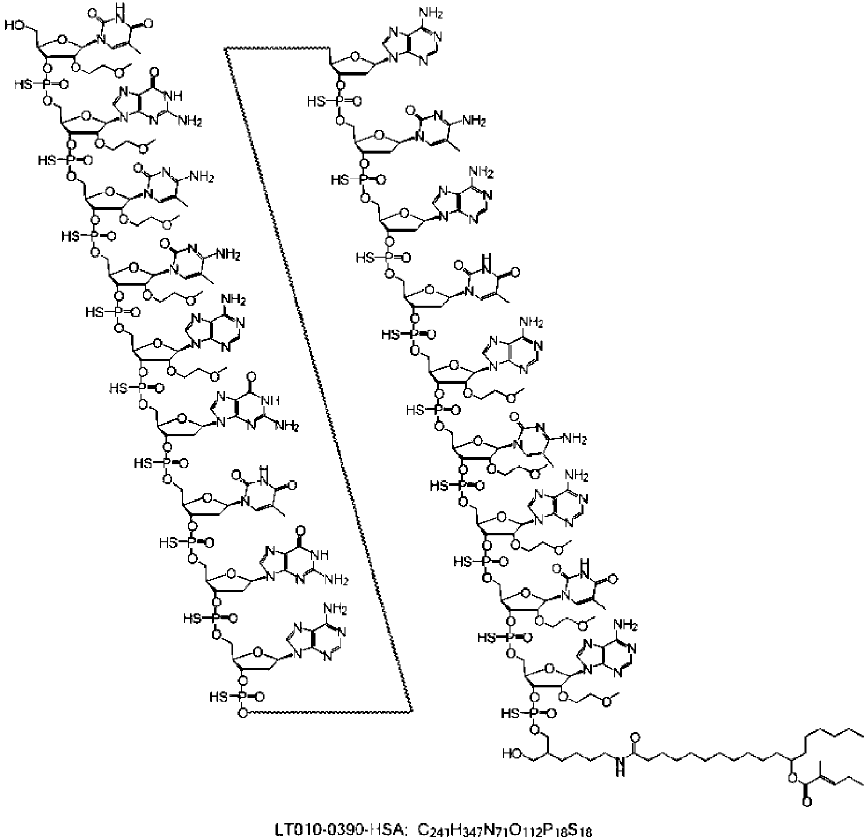
      LT-0390 Na : 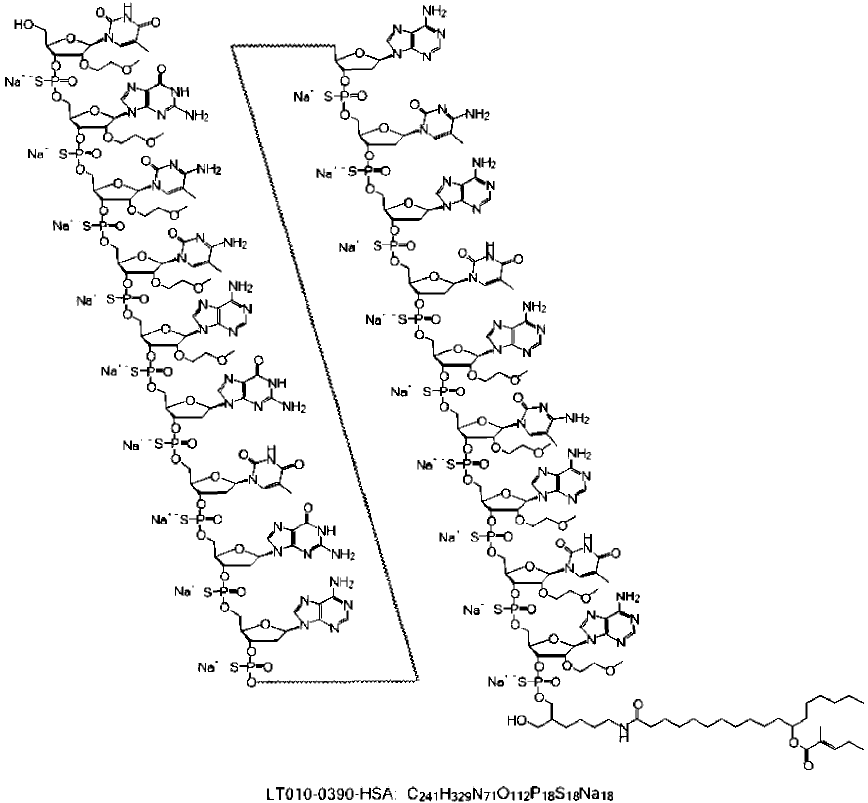
      LT-0390 S⁻: 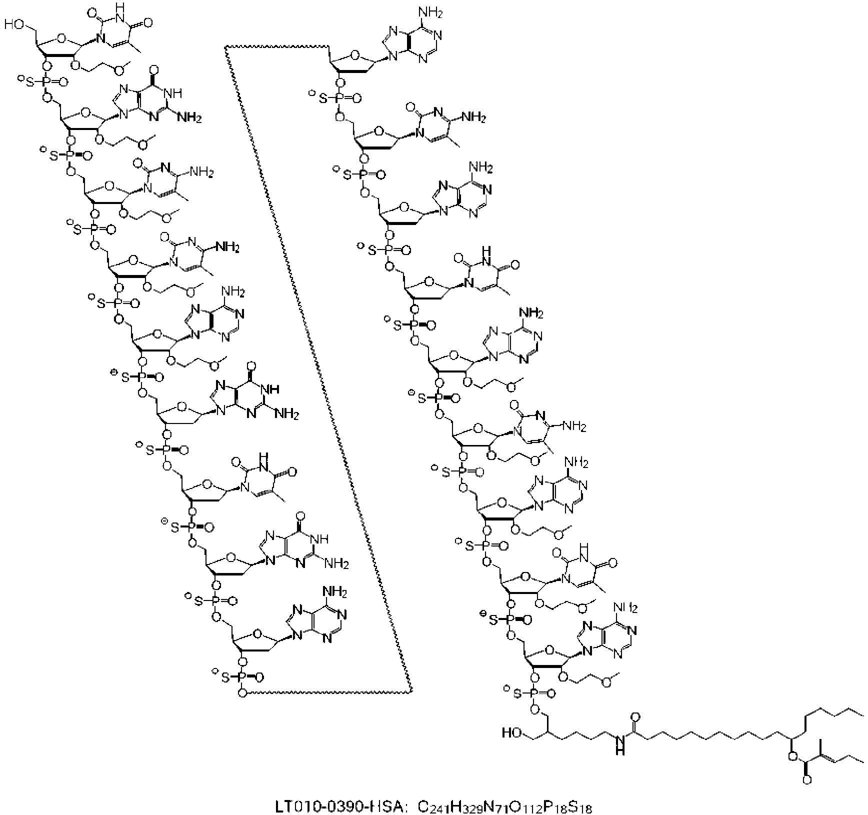
(2) LT-0449 (ASO ID. 289):
   T*G*CCA*g*t*g*a*a*c*a*t*ACA*T*A*HSA, modified as follows:
   *: phosphorothioate inter-nucleoside linkage;
   A, G, C, T: 2'-O-methoxyethyl; (2'-MOE);
   a, g, c, t: 2'-deoxy;
   all C or c base is 5-methyl-modified cytosine;
   HSA is: 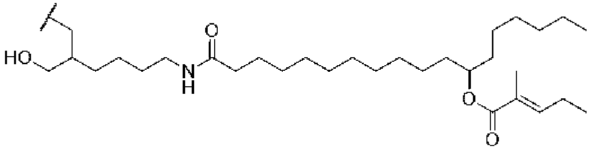
   The APIs can be hydrogen type (H type), sodium salt type (Na type), or S⁻ type, with structures as shown below:
      LT-0449 H : 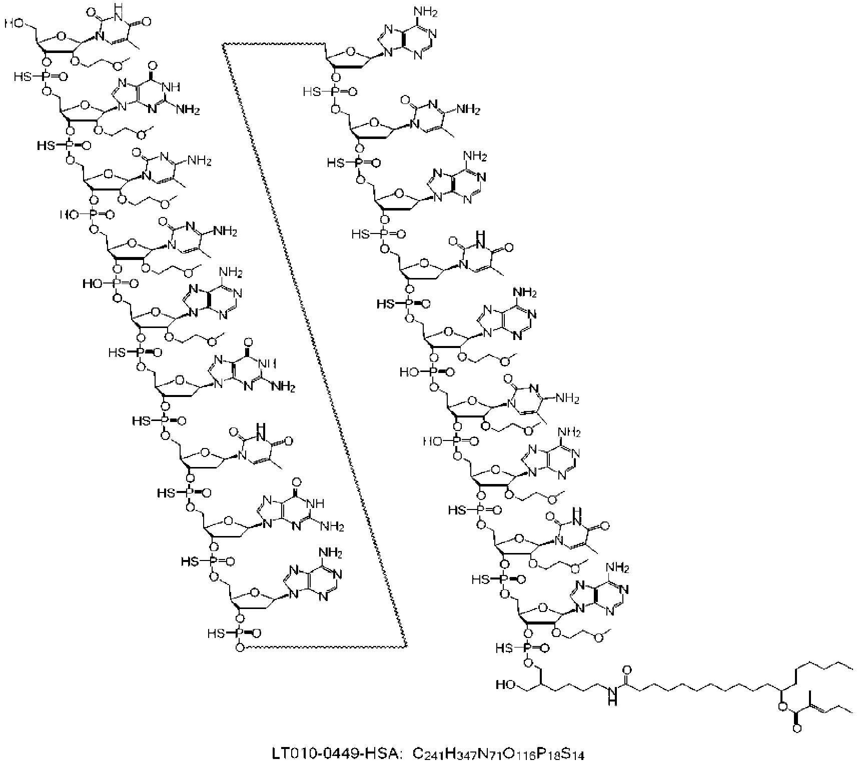
      LT-0449 Na : 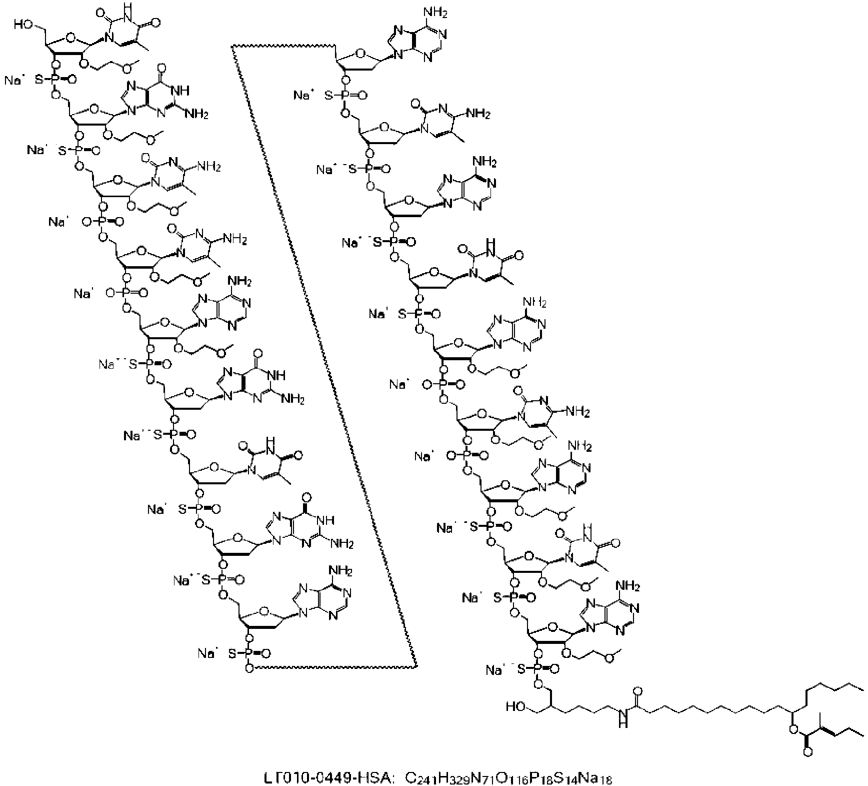
      LT-0449 S⁻ : 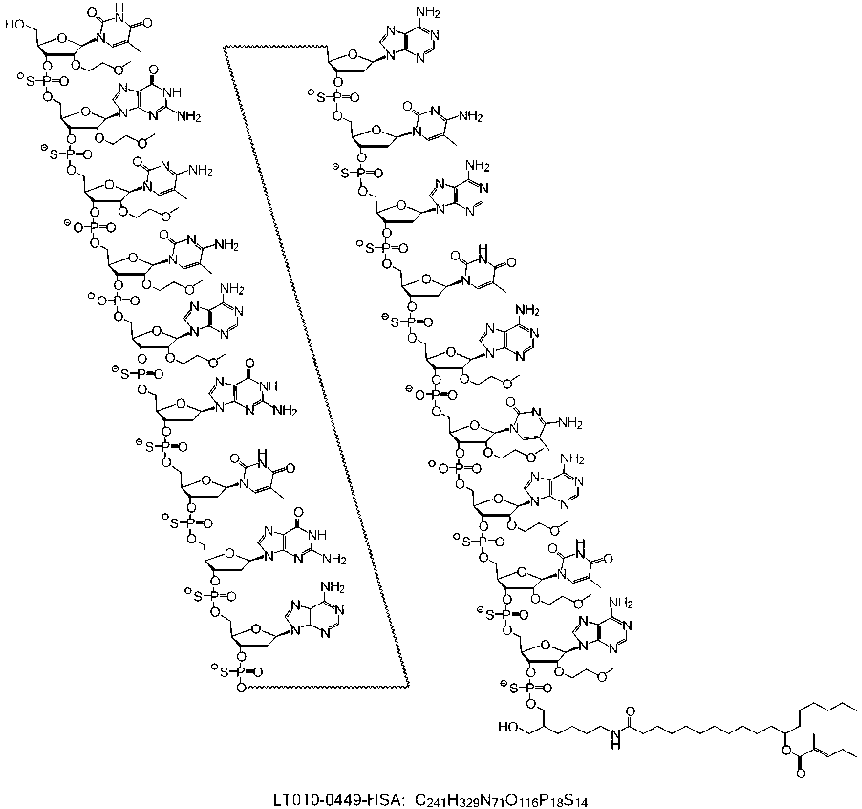
(3) LT-0410 (ASO ID. 204) :
   G*G*A*A*A*g*t*t*g*t*a*g*t*a*g*T*C*G*C*G*RA, modified as follows:
   *: phosphorothioate inter-nucleoside linkage;
   A, G, C, T: 2'-O-methoxyethyl; (2'-MOE);
   a, g, c, t: 2'-deoxy;
   all C base or c base is 5-methyl-modified cytosine;
   RA is: 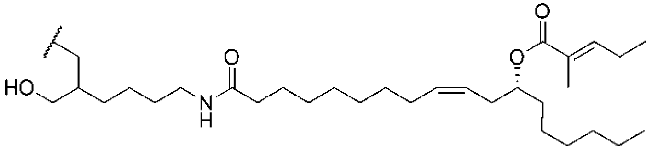
   LT-0410 H : 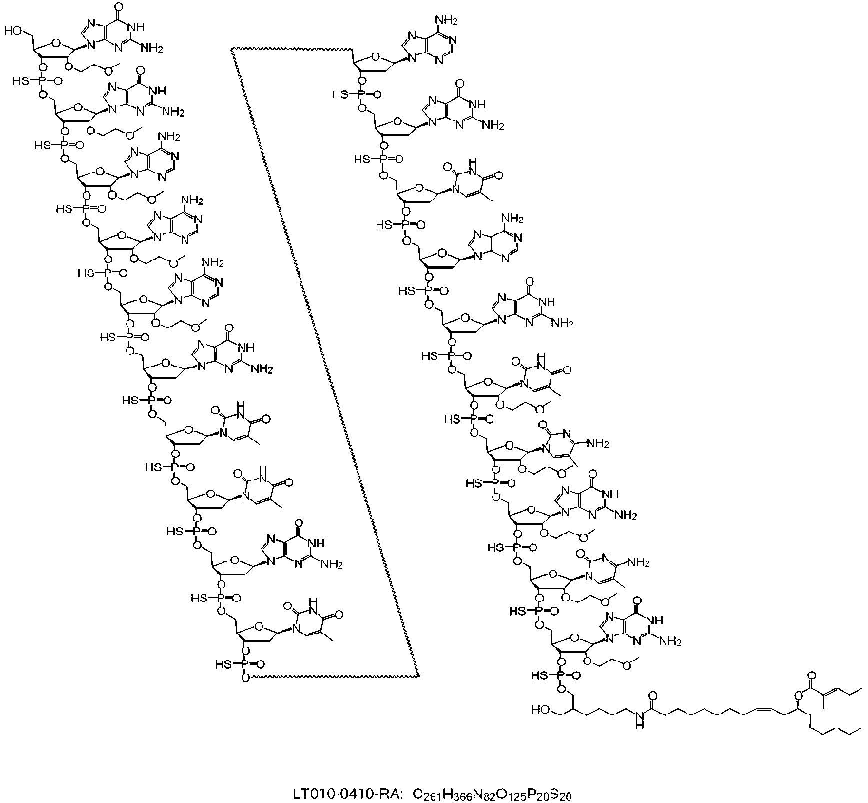
   LT-0410 Na : 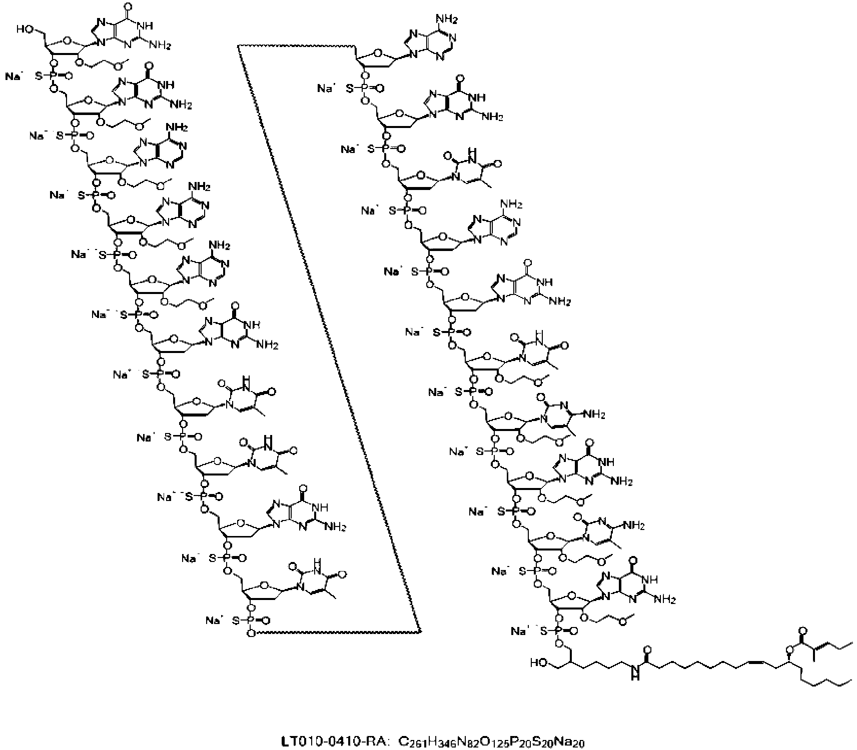
   LT-0410 S⁻ :

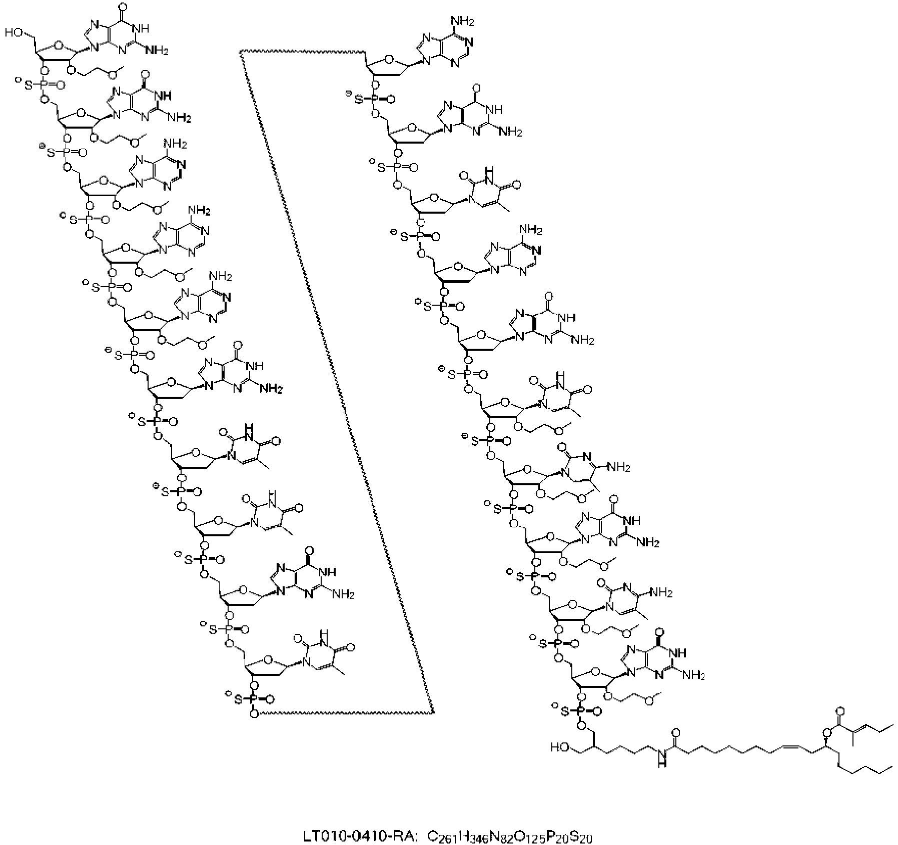

Finally, it should be noted that the above embodiments are only provided to assist those skilled in the art in understanding the present disclosure, and are not intended to limit the scope of the disclosure.

## Claims

1. A group of antisense oligonucleotides (ASO) that specifically inhibit the androgen receptor (AR), wherein the ASO are 14 to 22 bases in length, the target gene is AR mRNA, and the ASO specifically hybridize to specific region of the target gene, wherein the starting point of said specific region is located at:
(1) 5'UTR region: 132-144, 164-167, 191-229, 266-298;
(2) Exon 1: 1199-1201, 1591-1610, 1892, 2209-2210, 2306, 2572-2574;
(3) Exon 2: 2777-2782;
(4) Exon 2-Exon 3: 2878-2882;
(5) Exon 3: 2981-2983;
(6) Exon 4: 3113-3116;
(7) Exon 5: 3397-3401;
(8) Exon 5-Exon 6: 3432-3443;
(9) Exon 6: 3464, 3505-3506;
(10) Exon 6-Exon 7: 3563-3573;
(11) Exon 7: 3612, 3614-3616, 3618/3619, 3627, 3629, 3631, 3633;
(12) Exon 8: 3749-3759, 3818-3863;
(13) 3'UTR region: 7346-7354, 7394, 8139, 8418-8423, 10358-10363, 10386-10425; wherein the AR target gene is designated as: ENST00000374690.9, comprises the sequence as shown in SEQ ID NO. 63.

2. The ASO according to claim 1, wherein the ASO comprises chemical modifications, preferably, the chemical modifications comprise: thiol groups on the phosphate ester bonds, methoxyethyl modification at the 2'-position of the base (2'-MOE modification), and 5-methyl modification of cytosine,
more preferably, the chemical modifications comprise: mono-sulfur modification of the phosphate bonds of all the nucleotides in the ASO sequence, 2'-MOE modification on the same number of 3 to 5 bases at both the 3' and 5' ends, respectively, and 5-methyl modification of all cytosines.

3. The ASO according to claim 1 or 2, wherein the ASO is selected from the oligonucleotides shown in any of the sequences listed in the table below,
| ID | SEQ ID NO | Sequence | Target Region startPos(+) | Target Region |
|---|---|---|---|---|
| 150 | 1 | TGGCGTGGTGCGTCCC | 134 | 5UTR EXON1 |
| 13 | 2 | TGGGGCTGGCGTGGTGCGTCCC | 134 | 5UTR EXON1 |
| 161 | 3 | CTGGCGTGGTGCGTCC | 135 | 5UTR EXON1 |
| 134 | 4 | GCTGGCGTGGTGCGTCC | 135 | 5UTR EXON1 |
| 162 | 5 | GCTGGCGTGGTGCGTC | 136 | 5UTR EXON1 |
| 157 | 6 | GGCTGGCGTGGTGCGT | 137 | 5UTR EXON1 |
| 18 | 7 | GGCTGGGGCTGGCGTGGTGCGT | 137 | 5UTR EXON1 |
| 169 | 8 | GGGCTGGCGTGGTGCG | 138 | 5UTR EXON1 |
| 178 | 9 | GGGGCTGGCGTGGTGC | 139 | 5UTR EXON1 |
| 180 | 10 | CTGGGGCTGGCGTGGT | 141 | 5UTR EXON1 |
| 106 | 11 | GGCTGGGGCTGGCGTGGT | 141 | 5UTR EXON1 |
| 182 | 12 | GCTGGGGCTGGCGTGG | 142 | 5UTR EXON1 |
| 181 | 13 | GGCTGGGGCTGGCGTG | 143 | 5UTR EXON1 |
| 183 | 14 | GGGCTGGGGCTGGCGT | 144 | 5UTR EXON1 |
| 153 | 15 | CGTTGGCTGTCGCTGG | 164 | 5UTR EXON1 |
| 89 | 16 | GGCGTTGGCTGTCGCTGG | 164 | 5UTR EXON1 |
| 131 | 17 | GCGTTGGCTGTCGCTGG | 164 | 5UTR EXON1 |
| 164 | 18 | GCGTTGGCTGTCGCTG | 165 | 5UTR EXON1 |
| 120 | 19 | GGCGTTGGCTGTCGCTG | 165 | 5UTR EXON1 |
| 111 | 20 | AGGCGTTGGCTGTCGCT | 166 | 5UTR EXON1 |
| 151 | 21 | AGGCGTTGGCTGTCGC | 167 | 5UTR EXON1 |
| 163 | 22 | GCTTCGAAGCCGCCGC | 191 | 5UTR EXON1 |
| 156 | 23 | GGCTTCGAAGCCGCCG | 192 | 5UTR EXON1 |
| 155 | 24 | CGGCTTCGAAGCCGCC | 193 | 5UTR EXON1 |
| 152 | 25 | GGGCGGCGGCTTCGAA | 199 | 5UTR EXON1 |
| 176 | 26 | AGGAAAGGGCAGCTCC | 215 | 5UTR EXON1 |
| 177 | 27 | GAGGAAAGGGCAGCTC | 216 | 5UTR EXON1 |
| 174 | 28 | CGAAGAGGAAAGGGCA | 220 | 5UTR EXON1 |
| 167 | 29 | CCGAAGAGGAAAGGGC | 221 | 5UTR EXON1 |
| 166 | 30 | ACCGAAGAGGAAAGGG | 222 | 5UTR EXON1 |
| 173 | 31 | CACCGAAGAGGAAAGG | 223 | 5UTR EXON1 |
| 165 | 32 | TCACCGAAGAGGAAAG | 224 | 5UTR EXON1 |
| 172 | 33 | TTCACCGAAGAGGAAA | 225 | 5UTR EXON1 |
| 94 | 34 | ACTTCACCGAAGAGGAAA | 225 | 5UTR EXON1 |
| 158 | 35 | CTTCACCGAAGAGGAA | 226 | 5UTR EXON1 |
| 159 | 36 | ACTTCACCGAAGAGGA | 227 | 5UTR EXON1 |
| 81 | 37 | AAACTTCACCGAAGAGGA | 227 | 5UTR EXON1 |
| 154 | 38 | AACTTCACCGAAGAGG | 228 | 5UTR EXON1 |
| 149 | 39 | AAACTTCACCGAAGAG | 229 | 5UTR EXON1 |
| 170 | 40 | ACTTTCCTTGCTTCCT | 267 | 5UTR EXON1 |
| 179 | 41 | CACTTTCCTTGCTTCC | 268 | 5UTR EXON1 |
| 175 | 42 | GGACAAAGGCAGCCGT | 297 | 5UTR EXON1 |
| 91 | 43 | GAGGACAAAGGCAGCCGT | 297 | 5UTR EXON1 |
| 168 | 44 | AGGACAAAGGCAGCCG | 298 | 5UTR EXON1 |
| 237 | 45 | CGCGCACGCTCTGGAACAGA | 1201 | EXON1 |
| 194 | 46 | GAAAGTTGTAGTAGTCGCGA | 2209 | EXON1 |
| 271 | 47 | AAGTTGTAGTAGTCGCGA | 2209 | EXON1 |
| 204 | 48 | GGAAAGTTGTAGTAGTCGCG | 2210 | EXON1 |
| 272 | 49 | AAAGTTGTAGTAGTCGCG | 2210 | EXON1 |
| 250 | 50 | TGCATGCGGTACTCATTGAA | 3437 | EXON5_EXON6 |
| 193 | 51 | GTGCATGCGGTACTCATTGA | 3438 | EXON5_EXON6 |
| 219 | 52 | TGCCAGTGAACATACATAAA | 7346 | EXON8 3UTR |
| 192 | 53 | GTGCCAGTGAACATACATAA | 7347 | EXON8 3UTR |
| 289 | 54 | TGCCAGTGAACATACATA | 7348 | EXON8 3UTR |
| 285 | 55 | TAGTGCCAGTGAACATAC | 7351 | EXON8 3UTR |
| 4 | 56 | TTTTTAGTGCCAGTGAACAT | 7353 | EXON8 3UTR |
| 300 | 57 | TTTTAGTGCCAGTGAACA | 7354 | EXON8 3UTR |
| 206 | 58 | GAGGCACTAATGCTTGCTCC | 8139 | EXON8 3UTR |
| 5 | 59 | AACAGAACACTAGCGCTTGG | 10358 | EXON8 3UTR |
| 3 | 60 | GAACAGAACACTAGCGCTTG | 10359 | EXON8 3UTR |
| 241 | 61 | AGAGAACAGAACACTAGCGC | 10362 | EXON8 3UTR |
| 280 | 62 | GAGAACAGAACACTAGCG | 10363 | EXON8 3UTR |
preferably, the chemical modifications comprise: thiol groups on the phosphate ester bonds, methoxyethyl modification at the 2'-position of the base (2'-MOE modification), and 5-methyl modification of cytosine,
more preferably, the chemical modifications comprise:
mono-sulfur modification of the phosphate bonds of all the nucleotides in the ASO sequence,
2'-MOE modification on the same number of 3 to 5 bases at both the 3' and 5' ends, respectively, and
5-methyl modification of all cytosines,
after the modification, the sequences motif of the ASO is 4-8-4, 4-9-4, 5-8-5, 5-10-5, and 5-12-5.

4. Following uses of the ASO or their modified form according to any one of claims 1-3:
(1) preparing a formulation to inhibit the expression of the AR protein;
(2) preparing a drug or pharmaceutical composition for treating androgen-induced hair loss; or
(3) preparing a drug or pharmaceutical composition for treating androgen-induced acne; or
(4) inhibiting the expression or activation of the AR protein; or
(5) treating diseases caused by over-expression or excessive activation of the AR protein, wherein the diseases include but are not limited to: tumors, alopecia, or acne caused by overexpression or excessive activation of the AR protein; preferably, the tumor is prostate cancer,
wherein the drug or drug composition comprises a therapeutically effective amount of the ASO or their modified form, together with the necessary pharmaceutical excipients or delivery carriers.

5. A pharmaceutical composition comprising the ASO or their modified form according to any one of claims 1-3, wherein the pharmaceutical composition comprises: a therapeutically effective amount of the ASO or their modified form, and necessary pharmaceutical excipients or delivery carriers, preferably, the pharmaceutical composition is a transdermal formulation.

6. A topical formulation comprising the ASO or their modified form according to any one of claims 1-3, wherein the formulation comprises:
(1) the ASO or their modified form: 1-50 mg/mL; preferably 2-20 mg/mL; more preferably 4-10 mg/mL;
(2) sodium 8-(2-hydroxybenzoylamino) octanoate (SNAC): 5-50 mg/mL, preferably 30 mg/mL;
(3) dimethyl isosorbide (DMI): 5-60 mg/mL, preferably 40 mg/mL;
(4) vitamin E polyethylene glycol succinate (TPGS): 2-20 mg/mL, preferably 10 mg/mL;
(5) vitamin C: 2-20 mg/mL, preferably 10 mg/mL

7. The topical formulation according to claim 6, wherein the modified form of the ASO are,
(1) LT-0390 (ASO ID. 289):
T*G*C*C*A*g*t*g*a*a*c*a*t*A*C*A*T*A*HSA, modified as follows:
*: phosphorothioate inter-nucleoside linkage;
A, G, C, T: 2'-O-methoxyethyl; (2'-MOE);
a, g, c, t: 2'-deoxy;
all C or c base is 5-methyl-modified cytosine;
HSA is: 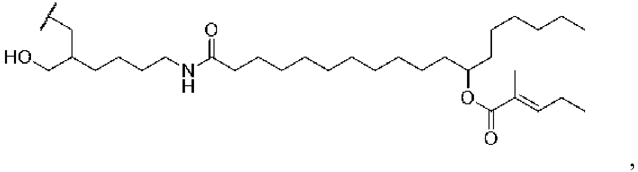 or
(2) LT-0449 (ASO ID. 289):
T*G*CCA*g*t*g*a*a*c*a*t*ACA*T*A*HSA, modified as follows:
*: phosphorothioate inter-nucleoside linkage;
A, G, C, T: 2'-O-methoxyethyl; (2'-MOE);
a, g, c, t: 2'-deoxy;
all C or c base is 5-methyl-modified cytosine;
HSA is: 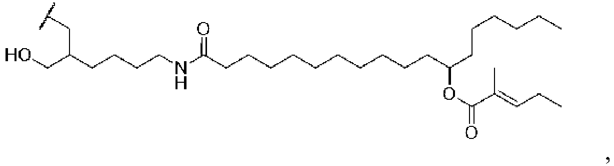 or
(3) LT-0410 (ASO ID. 204) :
G*G*A*A*A*g*t*t*g*t*a*g*t*a*g*T*C*G*C*G*RA, modified as follows:
*: phosphorothioate inter-nucleoside linkage;
A, G, C, T: 2'-O-methoxyethyl; (2'-MOE);
a, g, t: 2'-deoxy;
all C base is 5-methyl-modified cytosine;
RA is:

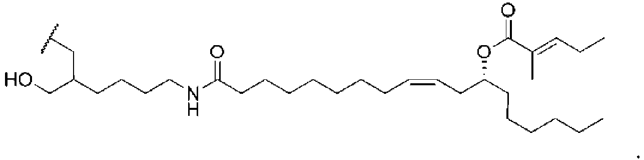

8. The topical formulation according to claim 7, wherein the modified form of the ASO are in the form of a hydrogen form, a sodium salt form, or an S⁻ form, with structures shown below:
LT-0390 H : 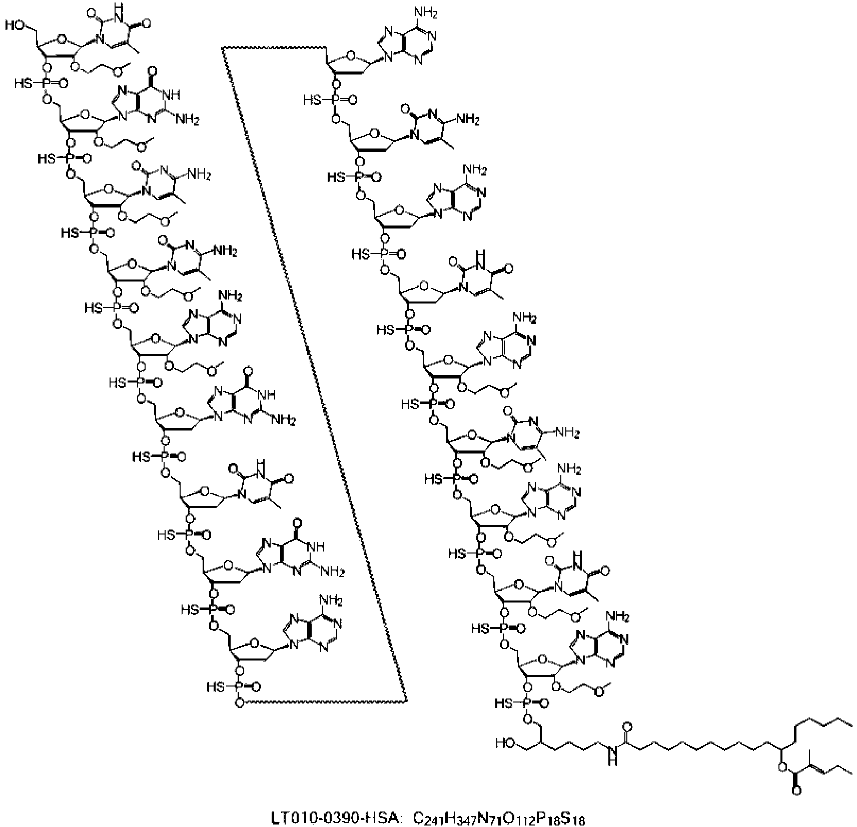
LT-0390 Na : 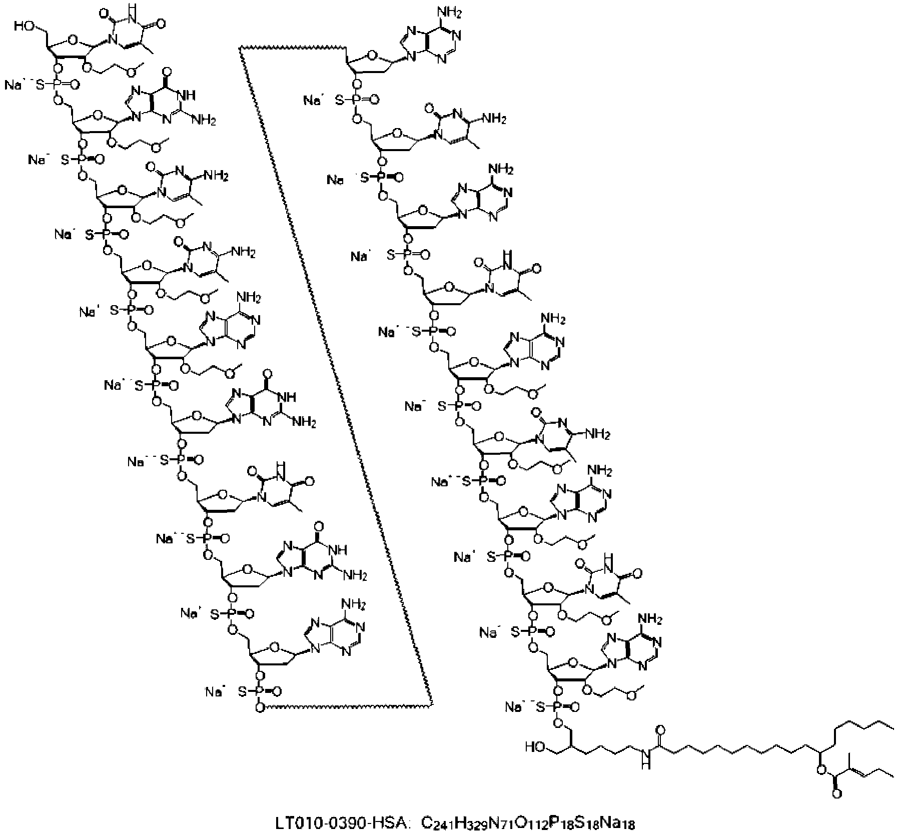
LT-0390 S⁻ : 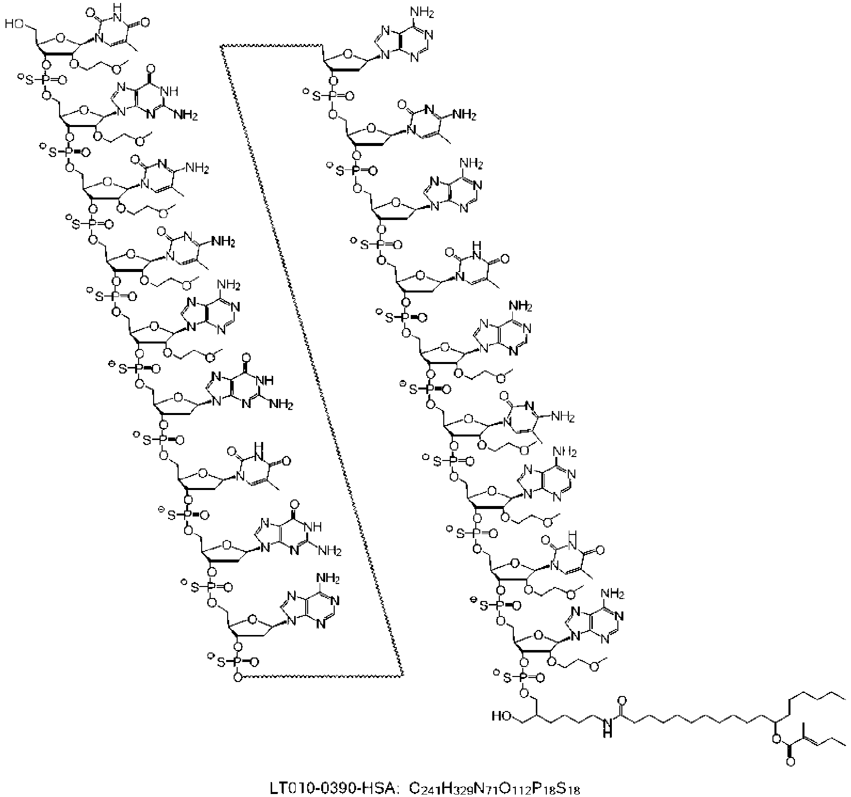
LT-0449 H : 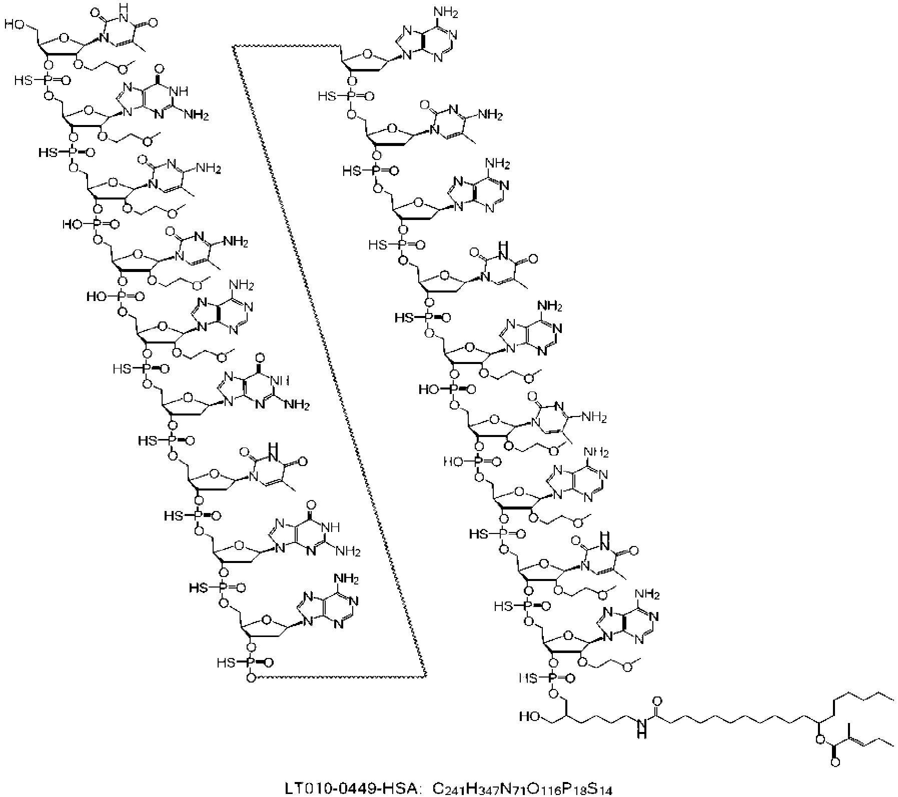
LT-0449 Na : 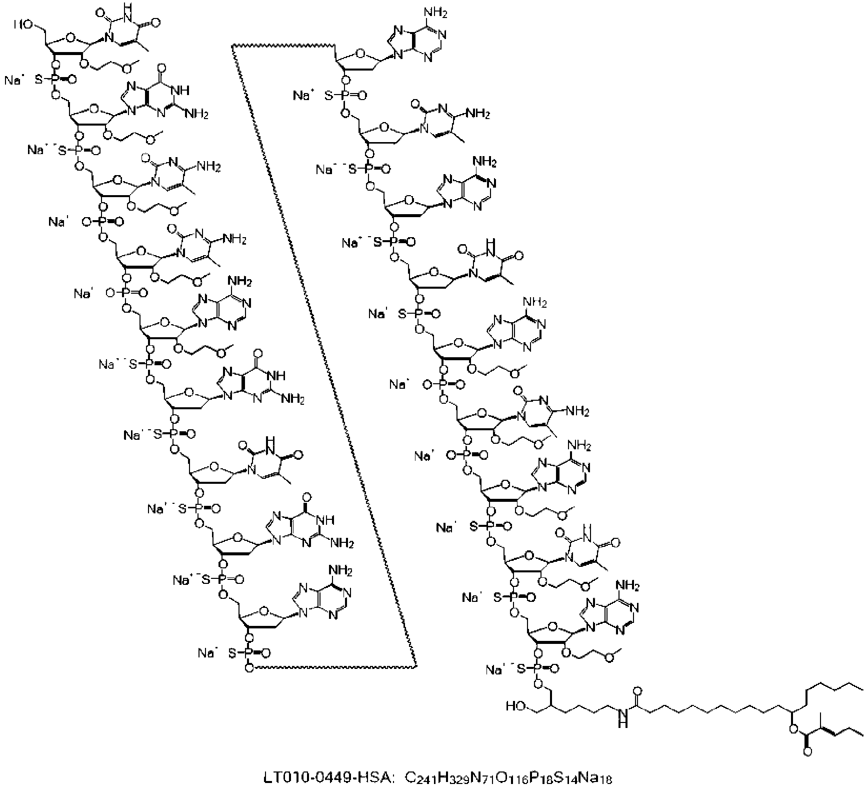
LT-0449 S^{- :} 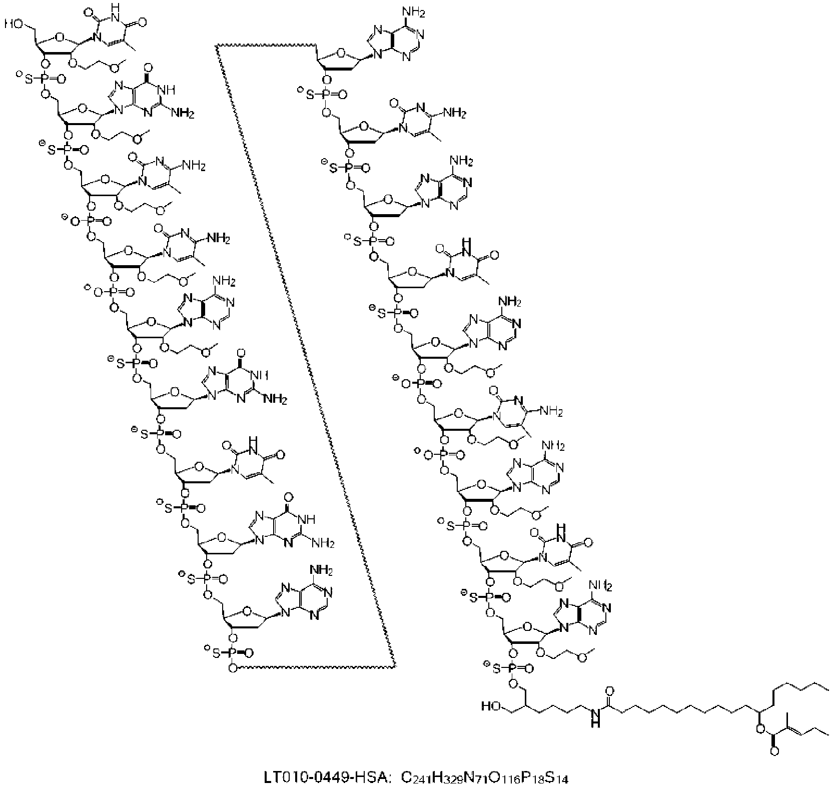
LT-0410 H : 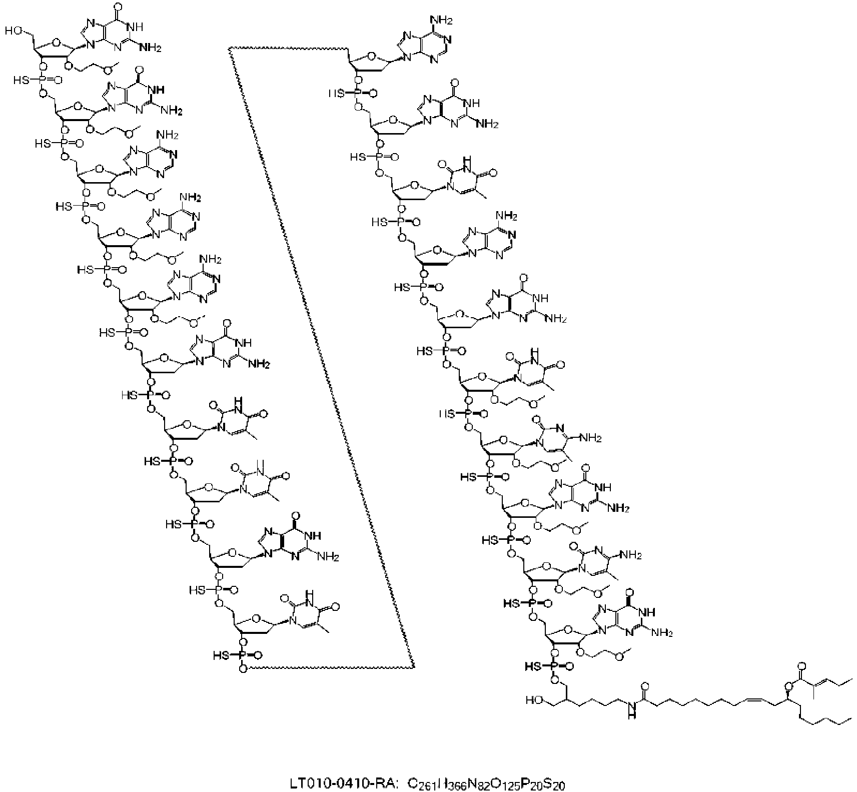
LT-0410 Na : 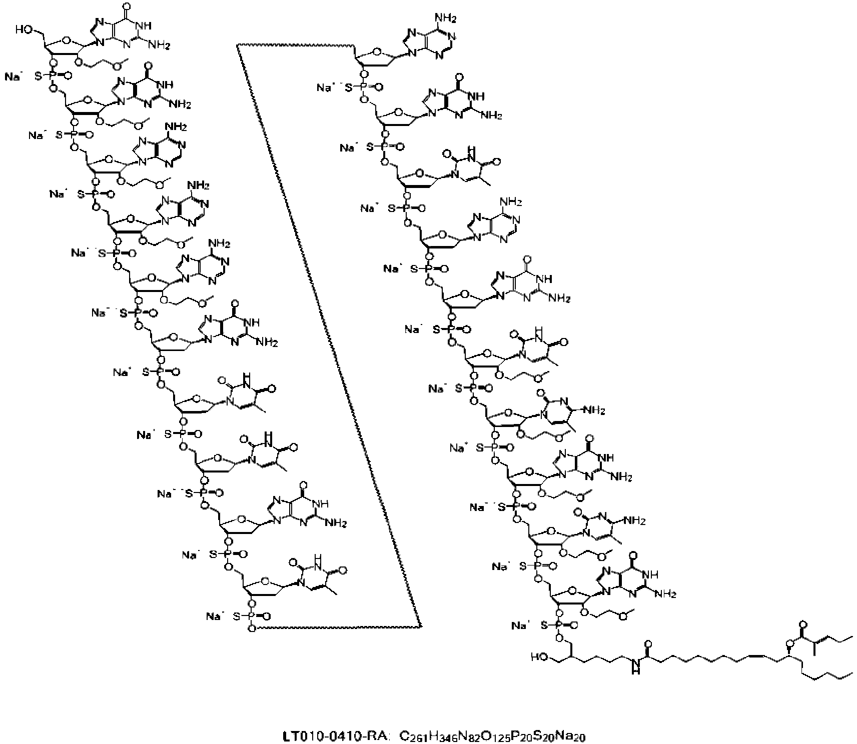
LT-0410 S⁻ :

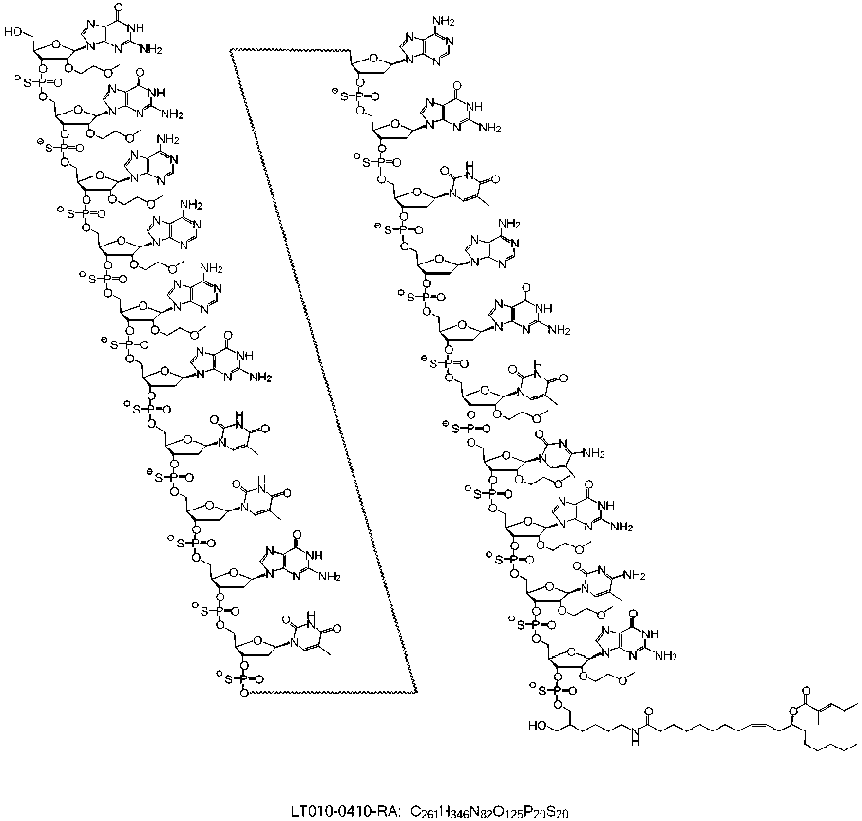

9. A method for preparing the topical formulation according to any one of claims 6-8, includes the following steps:
(1) oxygen equilibration: take purified water and equilibrate the dissolved oxygen in the water by purging it with nitrogen at a pressure of 0.1-0.2 MPa;
(2) dissolving excipients and adjusting pH: add SNAC, DMI, vitamin C, and TPGS to an appropriate amount of oxygen-equilibrated water and stir until dissolved; adjust the pH to 7.5-9.0, and re-inject nitrogen to re-equilibrate the dissolved oxygen;
(3) dissolving of the API: add the API (the ASO or its modified form), dissolve it and make up the volume with oxygen-equilibrated water; after making up the volume, nitrogen gas is introduced again to re-equilibrate the dissolved oxygen, resulting in the ASO/ASO-modified form topical formulation.

10. The method according to claim 9, wherein the method further comprises,
(4) filling and labeling: transfer the formulation to borosilicate glass vials, purge with nitrogen, seal with a rubber stopper and aluminum cap, crimp the cap, and apply a label.

11. Following uses of the topical formulation according to any one of claims 6-8,
(1) preparing a drug or pharmaceutical composition for treating male pattern hair loss; or
(2) preparing a drug or pharmaceutical composition for treating acne; or
(3) treating hair loss or acne caused by the overexpression or excessive activation of the AR protein.
